(19) Europäisches Patentamt / European Patent Office / Office européen des brevets

(11) **EP 4 260 875 A1**

(12) **EUROPEAN PATENT APPLICATION**

(43) Date of publication:
**18.10.2023 Bulletin 2023/42**

(21) Application number: **22305559.1**

(22) Date of filing: **15.04.2022**

(51) International Patent Classification (IPC):
***A61K 47/60*** $^{(2017.01)}$ ***A61K 47/64*** $^{(2017.01)}$
***A61P 35/00*** $^{(2006.01)}$

(52) Cooperative Patent Classification (CPC):
**A61K 47/60; A61K 33/26; A61K 47/645; A61P 35/00; B01J 31/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Feroscan**
  **92100 Boulogne-Billancourt (FR)**
- **UNIVERSITE D'ANGERS**
  **49000 Angers (FR)**

(72) Inventors:
- **LEPELTIER, Elise**
  **49000 ANGERS (FR)**
- **GUYON, Lena**
  **22700 PERROS GUIREC (FR)**
- **LADAYCIA, Abdallah**
  **49100 ANGERS (FR)**
- **PASSIRANI, Catherine**
  **49170 SAVENNIERES (FR)**
- **JAOUEN, Gérard**
  **94230 CACHAN (FR)**

(74) Representative: **Regimbeau**
**20, rue de Chazelles**
**75847 Paris Cedex 17 (FR)**

(54) **SELF-ASSEMBLIES OF FERROCIFEN CONJUGATES FOR LUNG CANCER TREATMENT**

(57) The present invention relates to a self-assembling product comprising a co-assembly of a CPP-ferrocifen conjugate, and a PEG-ferrocifen conjugate, wherein:

- the ferrocifen is a compound of the following formula (I):

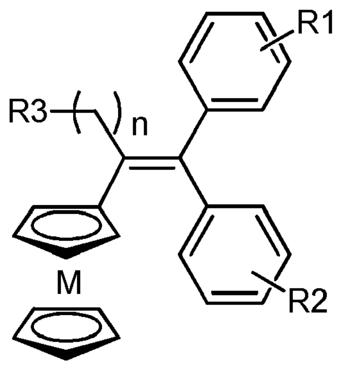

(I)

or a pharmaceutically acceptable salt or solvate thereof,

- CPP is a hydrophilic cell-penetrating peptide, covalently bonded to the R3 group of the ferrocifen by means of an amide (-C(O)NH-) bond; and
- PEG is a polyethylene glycol chain, covalently bonded to the R3 group of the ferrocifen by means of an ester (-C(O)O-) bond;

and wherein the CPP-ferrocifen / PEG-ferrocifen molar ratio is advantageously comprised between 100 / 1 and 1/1.

The present invention also relates to said self-assembling product for use as a drug, notably in the treatment of cancer, in a particular of lung cancer.

EP 4 260 875 A1

## Description

The present invention relates to a self-assembling product comprising a co-assembly of two conjugates of an anticancer drug.

Lung carcinoma is the major cause of cancer-related death worldwide, more particularly the non-small cell lung cancers (NSCLCs), accounting for about 85% of all lung cancers diagnosis. The chemotherapy treatment currently used consists of a combination of drugs: usually, cisplatin or carboplatin are used in combination with vinorelbine, gemcitabine, paclitaxel, docetaxel or pemetrexed. Despite continuous improvements in cancer treatments, the prognosis is poor, the efficacy is moderated and there are severe side effects. Thus, the development of new approaches is needed.

In the last decade, numerous nanocarriers have been designed as a transport module for anticancer drugs. Among them, self-assemblies formed by peptides and more particularly cell-penetrating peptides (CPP) have recently emerged as promising systems for controlled delivery of drug [Guyon et al., Nano Res. 2018, 11 (5), 2315-2335]. In fact, peptides have attractive properties due to their simple structure, biocompatibility and chemical diversity. In addition, CPP have the ability to cross cell membranes and enhance the intracellular delivery of conjugated cargos. The CPP are usually short peptides, containing a high relative abundance of positively charged amino acids such as arginine and/or lysine [Lindgren et al., Trends Pharmacol. Sci. 2000, 21 (3), 99-103.]. Nowadays, two main strategies for formulating drug loaded nanostructures formed by peptides can be used. The first one is to physically encapsulate drug into nanostructures formed of peptides. The second one is to covalently link the peptide to a drug, in order to form peptide-drug conjugates. The amphiphilic conjugates could potentially then self-assemble into nanostructures. This last strategy allows an increase of the drug loading, compared to the drug-encapsulated peptide nanoparticles and allows to avoid a potential toxicity brought by the excipients from the nanoparticle.

Over the past few years, Pr. Jaouen and his team have developed potent bioorganometalic complexes for anticancer application. Their chemistry is based on the coupling between a ferrocene moiety and a hydroxytamoxifen molecule in order to form ferrocifen molecules [Jaouen et al., Chem. Soc. Rev. 2015, 44 (24), 8802-8817]. The mechanism of action is related to the redox features of Fe through the reversible $Fe^{II}/Fe^{III}$ oxidation, leading to the production of ROS species and to a quinone methide, known to perform macromolecular interactions with thioredoxin reductase system, glutathione or DNA. Interestingly, ferrocifens are able to act by senescence at low concentrations and by apoptosis or Fenton reaction with increasing concentrations [Vessières et al., J. Inorg. Biochem. 2010, 104 (5), 503-511]. Moreover, ferrocifens have shown impressive *in vitro* biological activities on the NCI-60 cancer cell line panel.

However, a major drawback of ferrocifens, as well as of other anticancer drugs, is their hydrophobicity, leading to very low blood solubility and bioavailability. There exists thus a need for a new platform to vectorize hydrophobic anticancer drugs such as ferrocifens.

The inventors have discovered that CPP-drug and PEG-drug conjugates self-assemble to form new nanocarriers, which appear to be very attractive for the efficient and targeted delivery of hydrophobic anticancer drugs such as ferrocifens into cancer cells, notably lung cancer cells, with good tolerance.

Hence, the present invention relates to a self-assembling product, comprising a co-assembly of:

- a CPP-drug conjugate, and
- a PEG-drug conjugate,

wherein the drug is an anticancer drug chosen from among ferrocifens.

According to a preferred embodiment, the CPP-drug / PEG-drug molar ratio is comprised between 100 / 1 and 1/1, preferably between 50/1 and 2/1, notably between 20/1 and 5/1, in particular between 15/1 and 7/1, more preferably between 12/1 and 8/1, for example, about 10/1.

The present invention relates more particularly to a self-assembling product comprising a co-assembly of:

- a CPP-ferrocifen conjugate, and
- a PEG-ferrocifen conjugate,

wherein the CPP-ferrocifen / PEG-ferrocifen molar ratio is advantageously comprised between 100 / 1 and 1/1, preferably between 50/1 and 2/1, notably between 20/1 and 5/1, in particular between 15/1 and 7/1, more preferably between 12/1 and 8/1, for example, 10/1.

Within the context of the present invention, the term "self-assembling product" is intended to mean a product able to self-assemble in an aqueous medium, either spontaneously or via kinetic trapping (e.g. nanoprecipitation), to form nanostructures, *i.e.* structures which are nano-sized.

In particular, the self-assembling product according to the invention selfassembles by nanoprecipitation to form nano-sized particles, commonly referred to as nanoparticles.

When used with reference to a given structure or material, the term "nano-sized" is intended to mean that said

structure or material has a characteristic dimension comprised between 1 and 100 nm, notably between 1 and 50 nm, in particular between 1 and 10 nm.

Within the context of the present invention, said characteristic dimension is a hydrodynamic diameter.

For the purpose of the present invention, "hydrodynamic diameter", or $D_H$, means the diameter of a solute in solution, said solute being assumed to be spherical, as defined by the following Stokes-Einstein equation:

$$D = \frac{k_B T}{6\pi\rho R_H}$$

where:

- D is the diffusion coefficient of the solute in the solvent (expressed in $m^2.s^{-1}$),
- $k_B$ is the Boltzmann constant, with $k_B = 1.3806 \cdot 10^{-23}\ J \cdot K^{-1}$,
- T is the temperature of the medium (expressed in K),
- $\rho$ is the viscosity of the medium (expressed in Pa.s, or equivalent in $kg.s^{-1}.m^{-1}$), and
- $R_H$ is the hydrodynamic radius ($R_H = D_H /2$) expressed in m.

It should be noted that the self-assembling product of the present invention comprises a co-assembly of two distinct drug conjugates, each of which being able to self-assemble when taken alone. Besides, the self-assembly of drug-containing conjugates creates the drug's own delivery system, which is nano-sized. The self-assembling product of the present invention thus amounts to a nanocarrier material.

The ferrocifen comprised in the self-assembling product according to the invention is a compound of the following formula (I):

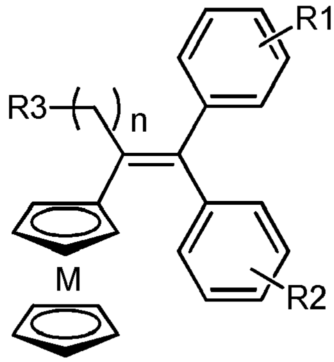

(I)

or a pharmaceutically acceptable salt or solvate thereof, in which:

- M is Fe (iron), Ru (ruthenium) or Os (osmium), preferably Fe,
- n is an integer comprised between 1 and 8,
- R1 and R2 are, independently from each other, H, $CF_3$, CN, $OR^4$ or $NR^5R^6$, and
- R3 is $CO_2R^7$, $OR^8$ or $NR^9R^{10}$,

wherein:

- $R^4$ is H, $(C_1\text{-}C_6)$alkyl, -CO-$(C_1\text{-}C_6)$alkyl or -$(CH_2)_m NR^{11}R^{12}$,
- $R^5$, $R^6$, $R^{11}$ and $R^{12}$ are, independently from one another, H, $(C_1\text{-}C_6)$alkyl or -CO-(Ci-$C_6$)alkyl,
- $R^7$ is H or $(C_1\text{-}C_6)$alkyl,
- $R^8$ is -CO-$(CH_2)_p CO_2R^{13}$
- $R^9$ is H or $(C_1\text{-}C_6)$alkyl,
- $R^{10}$ is -CO-$(CH_2)_q CO_2R^{14}$,
- $R^{13}$ and $R^{14}$ are, independently from one another, H or $(C_1\text{-}C_6)$alkyl,
- m, p and q are, independently from one another, integers comprised between 1 and 8.

In the present invention, "pharmaceutically acceptable" should be understood as designating what is useful in the preparation of a pharmaceutical composition, what is generally safe, non-toxic and neither biologically nor otherwise

undesired, and what is acceptable both for veterinary use and human pharmaceutics.

The term "pharmaceutically acceptable salt or solvate" is intended to mean, in the framework of the present invention, a salt or solvate of a compound which is pharmaceutically acceptable, as defined above, and which possesses the pharmacological activity of the corresponding compound.

The pharmaceutically acceptable salts comprise:

(1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and

(2) salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

Acceptable solvates for the therapeutic use of the compounds of the present invention include conventional solvates such as those formed during the last step of the preparation of the compounds of the invention due to the presence of solvents. As an example, mention may be made of solvates due to the presence of water (these solvates are also called hydrates) or ethanol.

The term "($C_1$-$C_6$)alkyl" should be understood as designating, within the meaning of the present invention, a saturated, linear or branched hydrocarbon group having from 1 to 6 carbon atoms, and advantageously from 1 to 4 carbon atoms, in particular methyl, ethyl, *n*-propyl, isopropyl, *n*-butyl, *iso*-butyl, sec-butyl and tert-butyl groups.

Within the context of the present invention, the term "CPP-drug conjugate" refers to a drug covalently bonded to a cell-penetrating peptide (CPP) by means of an amide (-C(O)NH-) bond.

In particular, the term "CPP-ferrocifen conjugate" refers to a ferrocifen as defined above covalently bonded to a CPP by means of an amide bond via its R3 group, which necessarily comprises a terminal ester (-$CO_2$-($C_1$-$C_6$)alkyl) or carboxylic acid (-$CO_2$H) function, allowing a peptide coupling with the amino terminal group of CPP.

For the purpose of the present invention, the term "cell-penetrating peptide", abbreviated as CPP, is intended to refer to any hydrophilic peptide or any peptide having a hydrophilic part in its sequence, known in the art to facilitate cellular intake and uptake of molecules, notably of anticancer drugs. The hydrophilic nature of the CPP allows to obtain an amphiphilic conjugate with the hydrophobic anticancer drug, which is thus able to self-assemble in an aqueous medium. In particular, the CPP is a polycationic peptide, comprising between 5 and 15, notably between 5 and 12, preferably between 6 and 9 positively charged amino acids, such as lysine and arginine, in particular arginine.

According to a particular embodiment of the present invention, the CPP is a polyarginine of the following formula ($Arg_{x/r}$):

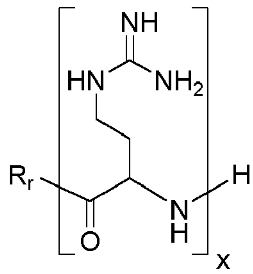

($Arg_{x/r}$),

wherein x is an integer comprised between 5 and 15, notably between 5 and 12, preferably between 6 and 9, and wherein $R_r$ is selected from the group consisting of -$NH_2$, -OH, -NH-($C_1$-$C_6$)alkyl-$NH_2$ and -O-($C_1$-$C_6$)alkyl-$NH_2$, preferably, $R_r$ is $NH_2$.

Within the context of the present invention, the term "PEG-drug conjugate" refers to a drug covalently bonded to a polyethylene glycol (PEG) chain by means of an ester (-C(O)O-) bond.

In particular, the term "PEG-ferrocifen conjugate" refers to a ferrocifen as defined above covalently bonded to a PEG moiety by means of an ester bond via its R3 group, which necessarily comprises a terminal ester (-$CO_2$-($C_1$-$C_6$)alkyl) or carboxylic acid (-$CO_2$H) function, allowing to form an ester bond with the terminal alcool (-OH) function of the PEG chain.

According to a particular embodiment of the present invention, the PEG moiety is of the following formula ($PEG_y$):

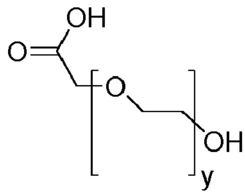

($PEG_y$),

wherein y is an integer comprised between 20 and 200, notably between 30 and 100, preferably between 40 and 50, for example, 44. When y = 44, the corresponding PEG chain has an average molecular weight of 2000 g/mol and is commonly referred to as PEG-2000.

According to a particular embodiment, the ferrocifen comprised in the self-assembling product according to the invention is a compound of formula (I) as defined above or a pharmaceutically acceptable salt or solvate thereof, wherein R1 and/or R2 are/is located in the para position on the phenyl ring, in particular R1 is located in the para position on the phenyl ring. Advantageously, R1 and R2 are located in the para position on the phenyl ring and then the ferrocifen has the following formula (Ia) or preferably (Ia-Fe):

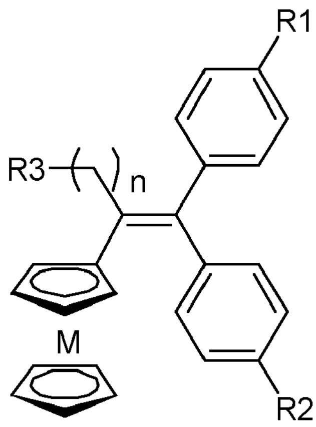

(Ia),

preferably

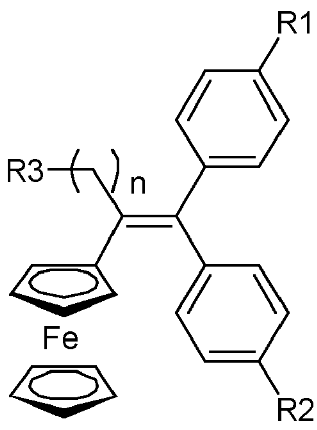

(Ia-Fe).

According to a particular embodiment, n is comprised between 2 and 6, notably between 2 and 5.

According to a particular embodiment, R1 and R2 are, independently from each other, H, $OR^4$ or $NR^5R^6$.

Advantageously, at least one of R1 and R2 is not a hydrogen, even more advantageously R1 is not a hydrogen, and notably both R1 and R2 are not a hydrogen.

In particular, at least one of R1 and R2 is $OR^4$ or $NR^5R^6$, and the other is notably H, $OR^4$ or $NR^5R^6$, preferably $OR^4$ or $NR^5R^6$.

In a preferred embodiment, at least one of R1 and R2 is $OR^4$, and the other is notably H or $OR^4$, preferably

$OR^4$, with $R^4$ as defined above and notably with $R^4$ = H.

According to a preferred embodiment, R1 and R2 are identical, preferably R1 and R2 are $OR^4$, more preferably OH.

According to a particular embodiment, R3 is $CO_2R^7$, $OR^8$ or $NR^9R^{10}$, wherein:

- $R^7$ is H,
- $R^8$ is $-CO-(CH_2)_pCO_2R^{13}$, wherein $R^{13}$ is H,
- $R^9$ is H or $(C_1-C_6)$alkyl, preferably H,
- $R^{10}$ is $-CO-(CH_2)_qCO_2R^{14}$, wherein $R^{14}$ is H,
- p and q are, independently from one another, integers comprised between 1 and 8, such as between 1 and 6, notably between 1 and 4, preferably between 2 and 3.

The ferrocifen comprised in the self-assembling product according to the present invention can be selected in particular from the following compounds, as well as pharmaceutically acceptable salts and solvates thereof:

| | |
|---|---|
| 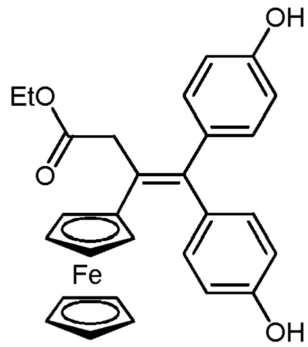<br>**P64** | 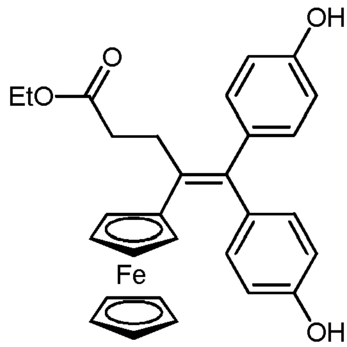<br>**P49** |
| 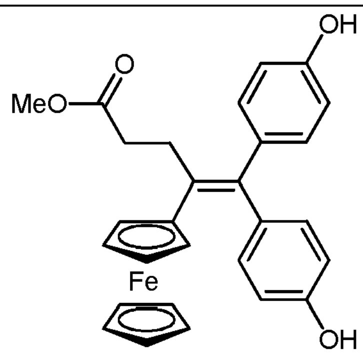<br>**P189** | 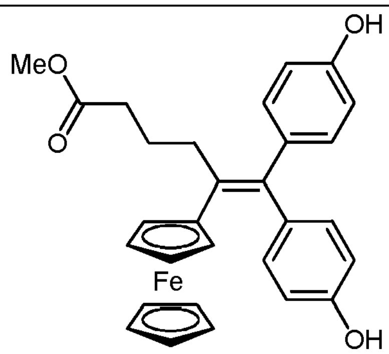<br>**P188** |
| 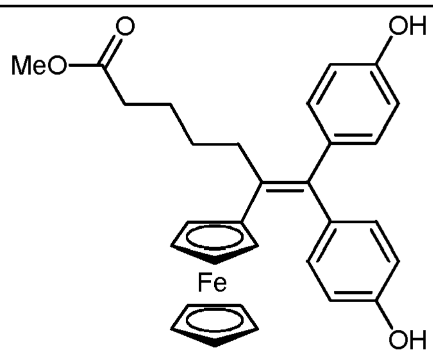<br>**P504** | 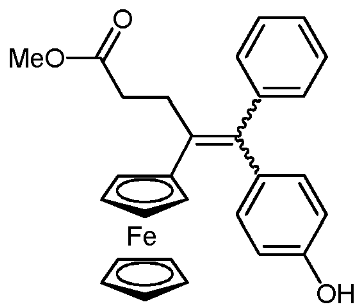<br>**P680** |
| 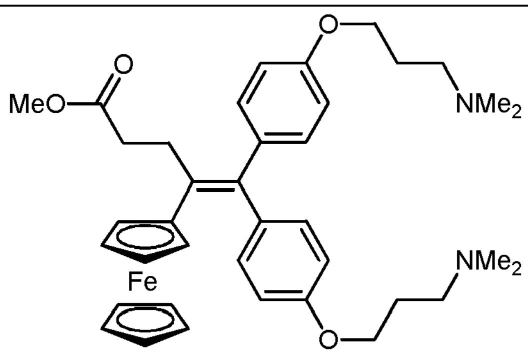<br>**P632** | 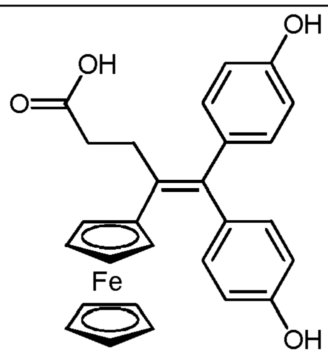<br>**P54** |

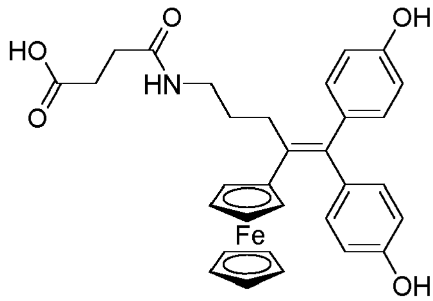

**P819**

In particular, it is selected from compounds P54 and P819, and the pharmaceutically acceptable salts and solvates thereof.

In a particular embodiment, the present invention relates to a self-assembling product comprising a co-assembly of:

- a CPP-ferrocifen conjugate, and
- a PEG-ferrocifen conjugate,

wherein:

- the CPP is a polycationic peptide, comprising between 5 and 15, notably between 5 and 12, preferably between 6 and 9 positively charged amino acids, such as lysine and arginine, in particular arginine, and preferably the CPP is of formula ($Arg_{x/r}$) as defined above;
- the PEG chain is of the following formula ($PEG_y$):

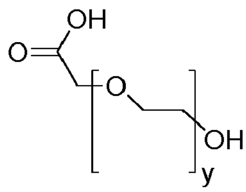 ($PEG_y$),

  wherein y is an integer comprised between 20 and 200, notably between 30 and 100, preferably between 40 and 50, for example, 44,
- the ferrocifen is as defined above,

and
wherein the CPP-ferrocifen / PEG-ferrocifen molar ratio is advantageously comprised between 100 / 1 and 1/1, preferably between 50/1 and 2/1, notably between 20/1 and 5/1, in particular between 15/1 and 7/1, more preferably between 12/1 and 8/1, for example, 10/1.

In a preferred embodiment, the CPP is a polyarginine of the following formula ($Arg_x$):

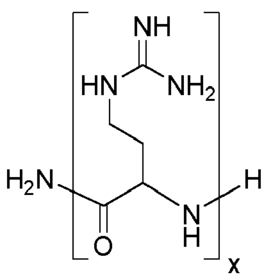 ($Arg_x$),

wherein x is an integer comprised between 5 and 15, notably between 5 and 12, preferably between 6 and 9.

In a more preferred embodiment, x is 9 and the CPP is thus $Arg_9$:

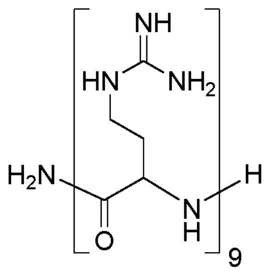

In a particular embodiment, the present invention relates to a self-assembling product comprising a co-assembly of:

- an $Arg_9$-ferrocifen conjugate, and
- a PEG-2000-ferrocifen conjugate,

wherein:

- the ferrocifen is as defined above, and
- the $Arg_9$-ferrocifen / PEG-2000-ferrocifen molar ratio is advantageously comprised between 100 / 1 and 1/1, preferably between 50/1 and 2/1, notably between 20/1 and 5/1, in particular between 15/1 and 7/1, more preferably between 12/1 and 8/1, for example, 10/1.

In particular, the ferrocifen is of formula (Ia-Fe):

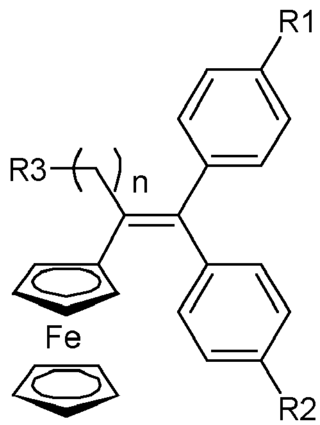

(Ia-Fe),

wherein:

- n is comprised between 2 and 6, notably between 2 and 5,
- R1 and R2 are identical and correspond to $OR^4$ or $NR^5R^6$, with $R^4$, $R^5$ and $R^6$ being as defined above, preferably R1 and R2 are $OR^4$, in particular OH, and
- R3 is $CO_2H$, $O\text{-}CO\text{-}(CH_2)_pCO_2H$ or $NH\text{-}CO\text{-}(CH_2)_qCO_2H$, wherein p, q is an integer comprised between 1 and 6, notably between 1 and 4, preferably between 2 and 3.

More particularly, the ferrocifen is selected from compounds P54 and P819, and the pharmaceutically acceptable salts and solvates thereof.

In a particular embodiment, the present invention relates to a self-assembling product comprising a co-assembly of:

- a CPP-ferrocifen conjugate, and
- a PEG-ferrocifen conjugate,

wherein:

- the CPP is of the above formula ($Arg_x$), wherein x is an integer comprised between 5 and 15, notably between 5 and 12, preferably between 6 and 9,
- the PEG chain is PEG-2000,
- the ferrocifen is compound P54 or a pharmaceutically acceptable salt or solvate thereof,

and
wherein the CPP-ferrocifen / PEG-ferrocifen molar ratio is comprised between 15/1 and 7/1, more preferably between 12/1 and 8/1, for example, 10/1.

In this particular embodiment, the CPP-ferrocifen has the following formula (Argx-P54):

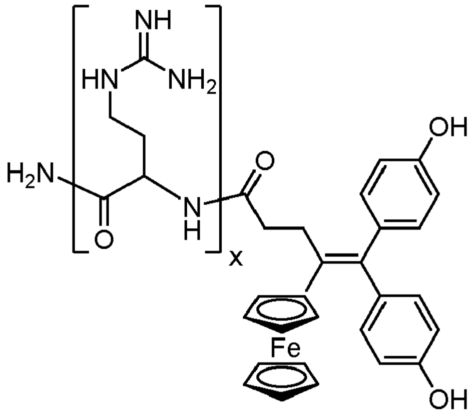

($Arg_x$-P54),

and the PEG-ferrocifen conjugate has the following formula (PEG-P54):

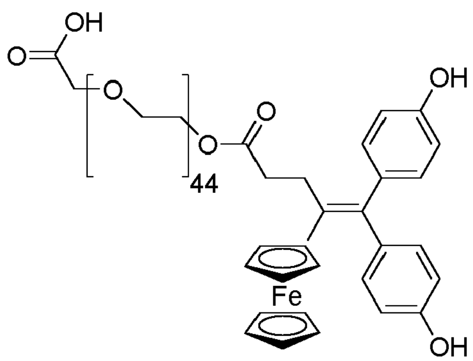

(PEG-P54).

The present invention is also directed to a self-assembling product as defined above for use as a drug, in particular in the treatment of cancer.

The present invention further relates to the use of a self-assembling product as defined above for the manufacture of a drug, especially intended for the treatment of cancer.

The present invention further relates to the use of a self-assembling product as defined above for the treatment of cancer.

The present invention further relates to a method for treating cancer comprising administering an effective amount of a self-assembling product as defined above to a patient in need thereof.

The cancer can be chosen among gliomas, melanomas, retinoblastomas, breast cancers (notably non-hormone-dependent breast cancers), prostate cancers, lung cancers (notably non-small-cell lung cancers), ovarian cancers, esophageal cancers, liver cancers, pancreatic cancers, head and neck cancers, colon cancers and kidney cancers.

In particular, the cancer is the lung cancer, notably the non-small-cell lung cancer. In this case, in order to improve the bioavailability and the delivery of the self-assembling product according to the invention, the administration route is preferably the intrapulmonary route via nebulization. Administrating the product by nebulization allows a drug delivery close to the cancer site, and so the opportunity to administrate a smaller quantity of the drug for the same or a better effect while decreasing its side effects. Also, administrating chemotherapy via nebulization would be more tolerable

by the patient than intravenous route and would allow outpatient care.

The present invention also provides a method for the preparation of a self-assembling product according to the invention by co-nanoprecipitation of the CPP-drug and PEG-drug conjugates, in particular of the CPP-ferrocifen and PEG-ferrocifen conjugates, comprising the steps of:

- solubilizing the conjugates in a polar organic solvent such as acetone or ethanol, preferably in a CPP-drug / PEG-drug molar ratio, in particular a CPP-ferrocifen / PEG-ferrocifen molar ratio, comprised between 100 /1 and 1/1, preferably between 50/1 and 2/1, notably between 20/1 and 5/1, in particular between 15/1 and 7/1, more preferably between 12/1 and 8/1, for example, 10/1;
- adding the obtained organic mixture dropwise to a volume of water under stirring, the water / polar organic solvent volume ratio being comprised between 10/1 and 2/1, for example between 8/1 and 2/1, notably between 6/1 and 3/1, in particular between 5/1 and 3/1, for example about 4/1; and
- evaporating the polar organic solvent.

Any polar organic solvent that solubilizes the conjugate, is miscible with water and can be easily evaporated may be used. In particular, the polar organic solvent is acetone or ethanol.

Adding dropwise the conjugates previously solubilized in the polar organic solvent to a volume of water causes the conjugates to form nanoparticles due to their amphiphilic nature, thus leading to the self-assembling product according to the invention after removal of the organic solvent.

After the evaporation of the solvent, it is preferable to leave the suspension under agitation for 10-15 minutes.

Advantageously, the final concentration of the CPP-drug conjugate in water is above its CAC value. Preferably, the final concentration of each conjugate is above its CAC value.

The conjugates may be prepared according to any method well-known to the skilled person to form an amide bond (e.g. peptide coupling) in the case of the CPP-drug conjugate or an ester bond in the case of the PEG-drug conjugate.

In particular, the CPP-ferrocifen and PEG-ferrocifen conjugates may be prepared as described in the examples.

Lastly, the present also relates to a PEG-ferrocifen conjugate, wherein:

- the PEG chain is of the following formula ($PEG_y$):

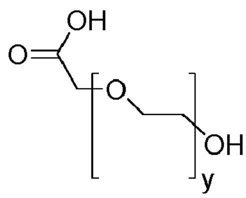

($PEG_y$),

wherein y is an integer comprised between 20 and 200, notably between 30 and 100, preferably between 40 and 50, for example, 44, and

- the ferrocifen is as previously described, and is covalently bonded to the PEG chain by means of an ester bond via its R3 group.

**BRIEF SUMMARY OF THE FIGURES**

Figures 1A, 1B, 1C and 1D correspond to the characterization data obtained for purified $Arg_9$-P54 by $^1H$ NMR in deuterated DMSO (A), LC-MS (B) and UPLC (C).

Figures 2A, 2B and 2C correspond to the characterization data obtained for purified PEG-P54 by MALDI-TOF (A), $^1H$ NMR in d6-DMSO (B) and UPLC (C).

Figure 3 represents the DOSY $^1H$ spectrum (left) of $Arg_9$-P54/PEG-P54 10/1 suspension in deuterated water at 25 °C after filtration through 0.2 μm and corresponding two components SCORE analysis (right).

Figure 4 shows the results obtained with CPP-P54 suspensions in a cell viability assay on NCI-H460 (n=4). The significance value was calculated using a Mann-Whitney test $*p \leq 0.05$; $**p \leq 0.01$; $***p \leq 0.005$. n=4. Statistical analysis was performed using P54 as reference.

Figure 5 shows the results obtained with $Arg_9$-P54/PEG-P54 suspensions at different molar ratios in a cell viability assay on NCI-H460 (n=3). The significance value was calculated using a Mann-Whitney test $*p \leq 0.05$; $**p \leq 0.01$; $***p \leq 0.005$. n=4. Statistical analysis was performed using P54 as reference.

Figure 6 represents the mice body weight evolution over 27 days for four groups: control group: n=8; group treated by self-assemblies of $Arg_9$-P54/PEG-P54 (10/1 molar ratio): n=6; group treated by self-assemblies of $Arg_9$-P54: n=6; group treated by self-assemblies of PEG-P54: n=7.

Figures 7A, 7B and 7C represent the tumor size doubling time of different groups: group treated by self-assemblies of $Arg_9$-P54/PEG-P54 (10/1 molar ratio) compared to the control group (A); group treated by self-assemblies of $Arg_9$-P54 compared to the control group (B); group treated by self-assemblies of PEG-P54 compared to the control group (C) (Control group: n=8, $Arg_9$-P54/PEG-P54: n=6, $Arg_9$-P54: n=6, PEG-P54: n=7; Shapiro-Wilk and Kolmogorov-Smirnov normality tests ; Student t test *$p \leq 0.05$; **$p \leq 0.01$; ***$p \leq 0.005$)

## EXAMPLES

The following abbreviations, commonly used in this field of art, are used in the following examples:

| | |
|---|---|
| ACN | : Acetonitrile |
| CAC | : Critical aggregation concentration |
| DIEA | : N,N-Diisopropylethylamine |
| DMF | : Dimethylformamide |
| DMSO | : Dimethylsulfoxyde |
| DOSY | : Diffusion-ordered spectroscopy |
| EDTA | : Ethylenediaminetetraacetic acid |
| FBS | : Fetal bovine serum |
| Fmoc | : Fluorenylmethoxycarbonyl |
| HBSS | : Hanks' Balanced Salt Solution |
| HBTU | : Hexafluorophosphate Benzotriazole Tetramethyl Uronium |
| HPLC | : High Performance Liquid Chromatography |
| $IC_{50}$ | : Half maximal inhibitory concentration |
| LC | : Liquid Chromatography |
| MALDI | : Matrix Assisted Laser Desorption Ionization |
| MBHA | : 4-Methylbenzhydrylamine |
| MS | : Mass Spectroscopy |
| NMR | : Nuclear Magnetic Resonance |
| PBS | : Phosphate buffered saline |
| MTT | : 3-(4,5-dimethylth-iazol-2-yl)-2,5-diphenyl tetrazolium |
| ROS | : reactive oxygen species |
| SD | : standard deviation |
| SPPS | : Solid-Phase Peptide Synthesis |

TFA : trifluoroacetic acid

TIS : Triisopropylsilane

TOF : Time of Flight

UPLC : Ultra Performance Liquid Chromatography

Materials

Polyethylene glycol (HO-PEG-COOH, MW 2 kDa) was purchased from Creative PEGWorks (USA). Dimethylformamide (DMF), trifluoroacetic acid (TFA) and acetonitrile (ACN) were obtained from Thermo Fisher Scientific (USA). Fmoc-Rink-Amid MBHA resin, all amino acids derivatives, hexafluorophosphate (HBTU), piperidine and diisopropylethylamine (DIEA) were purchased from Iris Biotech GmbH (Germany). Phenol was collected from Merck KGaA (Germany). Diethyl ether, LC-MS grade water and ULC/MS grade ACN were purchased from Biosolve (France). Triisopropylsilane (TIS) was obtained from Acros Organics. Deionized water was obtained from a Milli-Q plus system (Merck-Millipore, Germany). Ferrocifens were provided by PSL Chim ParisTech (France). Formic acid, acetone and pyrene were obtained from Sigma-Aldrich (France).

A549, NCI-H460 and BEAS-2B (ATCC® CRL9609™) cell lines were obtained from the American Type Culture Collection (USA). Roswell Park Memorial Institute 1640 (RPMI) medium was purchased from LGC Standards (France). BEGMTM Bronchial Epithelial BulletKitTM containing BEBMTM Bronchial Epithelial Cell Growth Basal Medium (CC-3171) and BEGMTM Bronchial Epithelial Cell Growth Medium SingleQuotsTM Supplements and Growth Factors (CC-4175) were purchased from Lonza (Switzerland). Fetal bovine serum (FBS), penicillin, streptomycin and phosphate-buffered saline (PBS) were obtained from Gibco (Fisher, France). Amphotericin B was obtained from PAA Cell Culture Company (Fisher, France). MTT (3-(4, 5- dimethylthiazol-2-yl)-2, 5-diphenyltetrazolium bromide)), Hanks' Balanced Salt Solution (HBSS) and dimethyl sulfoxide (DMSO) were purchased from Sigma-Aldrich (USA). Trypsin-EDTA 10X was obtained from Biowest (France).

**1. Conjugates synthesis and characterization**

Two different ferrocifens, P54 and P819, were used to synthesize corresponding CPP and PEG conjugates.

| | |
|---|---|
| 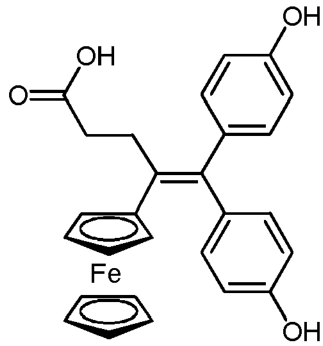 **P54** | 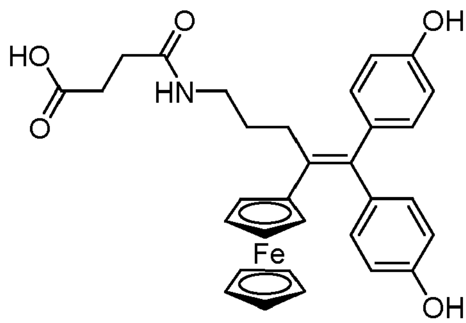 **P819** |

**1.1. Synthesis and purification**

1.1.1. CPP-ferrocifen conjugates

In order to synthesize the CPP-ferrocifen conjugates ($Arg_6$-P54, $Arg_7$-P54, $Arg_8$-P54, $Arg_9$-P54 and $Arg_9$-P819) CPP were covalently linked to ferrocifen moiety through an amide bond during the solid phase peptide synthesis, according to the following protocol (Guyon et al., J. Phys. Chem. Lett. 2019, 10, 6613-3320):

• $1^{st}$ step: SPPS (Merrifield synthesis) via a Fmoc strategy.

The MHBA resin substituted by $NH_2$ amine groups is used as solid support. The synthesis is carried out with

a peptide synthesizer. The reaction proceeds essentially in two steps:

- a deprotection step, using piperidine as deprotecting agent, and
- a coupling step in basic medium (DIEA), which requires activating the carboxylic acid function of the amino acid with a coupling agent (HBTU).

These steps are repeated in a cycle until the desired peptide chain is obtained. At the end of the synthesis, the resin is recovered in an adapted syringe.

• 2nd step: coupling of ferrocifen (P54 or P819) to the peptide on the N-terminus.

This coupling reaction is carried out in the syringe, under stirring for 2 h at 37 °C, using the following coupling solution: 2 eq. ferrocifen, 2 eq. HBTU, 3 eq. DIEA in DMF.

• 3rd step: cleaving of conjugate

After drying the resin in a desiccator, the conjugate is cleaved from the resin, recovered in solution and lyophilized. The cleavage reaction is carried out in the syringe under stirring for 2 h at 37 °C, using the following cleavage solution (cocktail K): TFA / phenol / water / TIS (88/5/5/2).

• 4th step: purification

Purification by semi-preparative HPLC, on a C18 column (XBridge Prep C18 5 µm OBD™ 30 × 250 mm). A gradient of two solvents (water/acetonitrile) is used to separate the different products of the synthesis, and thus isolate the fraction that corresponds to the purified conjugate.

1.1.2. <u>PEG-ferrocifen conjugates</u>

The ferrocifen molecule was conjugated with the PEG moiety using the same strategy than for CPP-ferrocifen.

In order to activate the carboxylic acid function, the ferrocifen (P54 or P819) (5 equiv.), containing a carboxylic acid function, was solubilized with HBTU (5 equiv.) in DMF. After 30 min, DIEA (10 equiv.) was added to the coupling mixture and immediately after, PEG-2000 in DMF (5 µM) was incorporated in the solution. The reaction was stopped after 2 h and DMF was removed by rotary evaporator.

The obtained oil was then purified by semi-preparative reversed phase highperformance liquid chromatography (RP-HPLC) using a Waters (France) instrument. Purification was performed at room temperature using SymmetryPrep C8 column (250 × 30 mm) with 7 µm particle and 100 Å pore size. Eluent

(A) was 0.1 % TFA in water while eluent (B) contained 0.1 % TFA in ACN. A gradient elution was used with a flow rate of 3 mL/min and an injection volume of 250 µL mL (Table 1). Peaks were detected at a wavelength of 450 nm (detection of the ferrocene part). The crude oil was solubilized in 30 % (A) and 70 % (B) at a concentration of 5 mg/mL. The sample was vortexed and sonicated prior to injection.

Table 1. Gradient elution of the RP-HPLC method for the purification of PEG-P54.

| Time (minutes) | Flow (mL/minutes) | Phase A (%) | Phase B (%) |
|---|---|---|---|
| 0 | 3 | 97 | 3 |
| 26 | 3 | 25 | 75 |
| 40 | 3 | 10 | 90 |
| 42 | 3 | 10 | 90 |
| 43 | 3 | 97 | 3 |
| 50 | 3 | 97 | 3 |
| Notes: Phase (A) contained 0.1 % TFA in water and phase (B) 0.1 % TFA in ACN | | | |

### 1.2. Characterization

1.2.1. CPP-ferrocifen conjugates

- After purification by semi-preparative HPLC, CPP-ferrocifen conjugate was analyzed by NMR, LC-MS (micromass Quattro micro ™) and UPLC (Acquity UPLC H class Bio) to identify it and determine its purity.
- $Arg_9$-P54 was obtained with a yield of 24 % and a purity of 94 %.

The $^1$H NMR spectrum in deuterated DMSO, LC-MC spectrum and UPLC chromatogram obtained for this conjugate are represented in Figures 1A, 1B, 1C and ID.

$Arg_6$-P54 (yield: 20%; purity: 88%), $Arg_7$-P54 (yield: 21%; purity: 89%), $Arg_8$-P54 (yield: 29%; purity: 98%) and $Arg_9$-P819 (yield: 25%; purity: 91%) were also obtained.

1.2.2. PEG-ferrocifen conjugates

- After purification by RP-HPLC, PEG-ferrocifen conjugate was analyzed by $^1$H NMR spectroscopy and mass spectrometry.

Nuclear magnetic resonance (NMR) spectroscopy: $^1$H NMR spectra were recorded in deuterated dimethyl sulfoxide at 400 MHz in the FT mode with a Bruker 500MHz AVANCE III HD spectrometer (Wissembourg, France) equilibrated at 25 °C. Spectra were analyzed using the software MestReNova®.

The $^1$H NMR spectrum in deuterated DMSO obtained for PEG-P54 is represented in Figure 2B.

Mass spectrometry (MS): Matrix-assisted laser-desorption ionization time-of-flight (MALDI-TOF) spectra was recorded by using a Bruker Biflex III spectrometer with a wavelength laser of 337 nm. 2-[(2E)-3-(4-tert-Butylphenyl)-2-methylprop-2-enylidene]malononitrile (DCTB) was used as a matrix. Positive ion spectra were acquired in linear mode, PEG-P54 was solubilized in dichloromethane prior to acquisition.

The MALDI-TOF spectrum of free PEG was compared to the PEG-P54 spectra (Fig. 2A). A shift of the PEG signal peaks confirmed that the coupling reaction was successful. - Moreover, UPLC experiment was performed to quantify the purity of the PEG-P54 and PEG-P819.

Ultra Performance Liquid Chromatography (UPLC): An UPLC-UV method was developed to quantify the purity of PEG-P54 and PEG-P819. The apparatus consisted of an UPLC Acquity H-Class Bio (Waters, France) composed of a quaternary solvent manager, a sample manager, a photo diode array detector and a column manager. The system was managed by Empower®3 software (Waters). The column used was an Acquity®UPLC BEH C18 100 × 2.1 mm, 1.7 μm (Waters). The mobile phase was composed of a mixture of 0.1 % TFA in water (A) and 0.1 % TFA in ACN. Flow rate was 0.2 mL/min and injection volume was set to 10 μL. Detection was performed at 450 nm. The purified product was dissolved in 95 % (A) and 5 % (B) at a concentration of 1 mg/mL.

Table 2. Gradient elution of the UPLC-UV method.

| Time (minutes) | Flow (mL/minute) | Phase A (%) | Phase B (%) |
|---|---|---|---|
| 0 | 0.2 | 95 | 5 |
| 5 | 0.2 | 78 | 22 |
| 10 | 0.2 | 78 | 22 |
| 15 | 0.2 | 0 | 100 |
| 20 | 0.2 | 0 | 100 |
| 21 | 0.2 | 95 | 5 |
| 25 | 0.2 | 95 | 5 |
| Notes: Phase (A) contained 0.1 % TFA in water and phase (B) 0.1 % TFA in ACN | | | |

- Finally, PEG-P54 was obtained with a yield of around 11 % and a purity of 90 % (Fig. 2C) and PEG-P819 with a

yield of 12 % and a purity of 91 %.

### 1.3. Critical aggregation concentration (CAC)

The critical aggregation concentration (CAC) was determined using pyrene as a fluorescent probe as reported in Guyon et al., J. Phys. Chem. Lett. 2019, 10, 6613-3320. For thus, 6 μL of pyrene stock solution in acetone (50 μM) was added into tubes. Then, acetone was evaporated in dark condition. Different suspension of the tested conjugates with a concentration ranging from 0.5 to 2000 μM were added into tubes and mixed overnight at 37°C in order to have a final pyrene concentration of 1 μM. After 30 min of equilibration at room temperature, a fluorescence spectrophotometer (Fluoromax-4, Horiba, Japan) was used to measure the fluorescence intensities of pyrene at an excitation wavelength of 336 nm. The emission spectra were recorded in the range 350-500 nm. The slit opening for the excitation was set at 1 nm and 3 nm for the emission. Intensity ratios of pyrene at 1372/1382 (11/13) were plotted against the log of the concentration and results were fitted by a Boltzmann-type sigmoid. The CAC value was determined as the first sharp decrease point.

The obtained values are reported in Table 3 below.

Table 3. CAC values.

| Compound | CAC (μg/mL) | CAC (μM) |
|---|---|---|
| Arg$_6$-P54 | 52 | 37 |
| Arg$_7$-P54 | 75 | 48 |
| Args-P54 | 170 | 99 |
| Arg$_9$-P54 | 210 | 113 |
| PEG-P54 | 553 | 243 |
| P54 | 325 | 649 |
| Arg$_9$-P819 | 413 | 211 |

## 2. <u>Self-assembly formulations and characterization</u>

### 2.1. Formulation protocol

• Nanoprecipitation of a single CPP-ferrocifen or PEG-ferrocifen conjugate

The formation of a self-assembly of a given conjugate is carried out by nanoprecipitation, which is performed using a solvent displacement method.

For this, the conjugate was solubilized in an adequate organic solvent (acetone for CPP-ferrocifen conjugate, ethanol for PEG-ferrocifen conjugate), and the obtained organic mixture was then added drop by drop to a volume of water (4 times the volume of the organic solvent) under agitation. The organic solvent was then evaporated via an argon flow under agitation.

The formulation process is performed at a concentration above the CAC value of the conjugate.

• Co-nanoprecipitation of CPP-ferrocifen/ PEG-ferrocifen

The same protocol was used to prepare co-assemblies of CPP-P54/PEG-P54 and CPP-P819/PEG-P819. For this, the conjugates were solubilized in the adequate organic solvent at different molar ratios.

- The following self-assemblies were obtained accordingly:

  - Arg$_6$-P54, Arg$_7$-P54, Arg$_8$-P54, Arg$_9$-P54 and Arg$_9$-P819;
  - PEG-P54;
  - Arg$_9$-P54 / PEG-P54 at 10/1, 2/1, 1/1 and 1/2 molar ratios; and
  - Arg$_9$-P819 / PEG-P819 at a 10/1 molar ratio.

### 2.2. Self-assembly characterization

- In order to determine the self-assembly diameters, the formulations were analyzed via NMR diffusometry (DOSY).

$^1$H NMR pulsed field gradient acquisition (PFG-NMR), also called diffusion ordered spectroscopy (DOSY), was performed on the self-assemblies (9 mg/mL) in deuterated water at 25 °C after a 200 nm filtration. A Bruker 500 MHz AVANCE III HD spectrometer (Wissembourg, France) equipped with a 5 mm-BBFO probe at the ASTRAL NMR facility from Angers University was used. In DOSY measurements, diffusion coefficients are estimated related to chemical shift by the observation of the exponential decay of the NMR signal due to the self-diffusion behaviour taking place between the two gradients of magnetic field.

In case of a mixture, the signal can be represented by the Laplace transform of a diffusion coefficient distribution, f(D), of species that contribute to the signal: *5(b)* = S0. ∫*f*(*D*). exp (-*D*. *b*) . *dD* , where b is called the diffusion weighted factor. b is expressed as: $b = \gamma^2.g^2.\delta^2.(\Delta - \frac{\delta}{3})$ , with g the gyromagnetic ratio, g the gradient intensity, $\delta$ the gradient duration and $\Delta$ the gradient separation. Diffusion experiments were performed using the stimulated echo sequence with longitudinal Eddy current delay (LED), *ledgp2s.* As the larger object diffusivity was not possible to estimate due to their small contribution, the acquisition parameters were chosen to optimally evaluate diffusion coefficients of the smaller objects. The diffusion time $\Delta$was 200 ms, the diffusion gradient duration $\delta$ was 2.4 ms, the gradient shape ramp was linear with 128 steps and the number of averages was 96. The gradient magnitude ranged from 5 to 95 % of the maximum gradient intensity (50 $G.cm^{-1}$). The relevant integrated domain of spectra signals was measured using TOPSPIN 3.6.1 (Bruker, Wissembourg), then analysed using the REPES ILT algorithm. - Apparent hydrodynamic diameters ($D_h$) were calculated from the estimated diffusion coefficient D according to Stokes-Einstein relation (using 1.10 mPa×s for $D_2O$ viscosity at 25 °C and 0.31 mPa×s for deuterated acetone viscosity at 25 °C). The obtained values are reported in Table 4 below.

Table 4. Apparent hydrodynamic diameters ($D_h$) values.

| Self-assembly | $D_h$ (nm) in $D_2O$ | $D_h$ (nm) in deuterated acetone |
|---|---|---|
| $Arg_6$-P54 | 2.3 nm | 1.6 nm |
| $Arg_9$-P54 | 4.1 nm | 0.9 nm |
| PEG-P54 | 4.1 nm | 0.7 nm |
| $Arg_9$-P54 / PEG-P54 (10/1) | 2.9 nm | - |
| $Arg_9$-P819 | 4.5 nm | 0.9 nm |
| $Arg_9$-P819 / PEG-P819 (10/1) | 4 . 4 nm | - |

The DOSY 1H spectrum obtained for the $Arg_9$-P54/PEG-P54 (10/1) suspension in deuterated water at 25 °C after filtration at 0.2 $\mu$m is represented in Figure 3.

More than 99% of the material was shown to self-assemble into 1.3 nm radius nanoparticles for $Arg_9$-P54/PEG-P54 (10/1) formulations.

### **3.** Biological activity of suspensions: ***in vitro* cell viability assays**

### 3.1. Methods

The toxicity of self-assemblies was compared to free ferrocifen P54 on the human lung adenocarcinoma cells (A549), human large-cell lung carcinoma (NCI-H460) and human lung epithelial cells (BEAS-2B). A549 and NCI-H460 cells were maintained in Roswell Park Memorial Institute 1640 (RPMI) medium containing 10 % fetal bovine serum (FBS) and 1 % antibiotics. BEAS-2B cells were maintained in Bronchial Epithelial Cell Growth Basal Medium with all the additives as recommended by the supplier. Each cell line was maintained in a humidified incubator with an atmosphere containing 5 % CO2 at 37°C. BEAS-2B cells were subcultured once a week, while A549 and NCI-H460 twice a week, using 0.25 % trypsin in EDTA (dilution 1:6 in HBSS). The cell viability was determined with a colorimetric assay using succinate dehydrogenase activity of viable cells by the reduction of the yellow-colored tetrazolium salt, 3-(4,5-dimethylthiazol-2-yl)-2,5-diphenyl tetrazolium bromide to a purple-colored formazan crystal (MTT assay). Briefly, cells were plated in 96-well plates at densities of 10x103 cells/well (A549), 10×103 cells/well (NCI-H460) and 20×103 cells/well (BEAS-2B). After 24 h, cells were treated for 24 h with different concentrations of the self-assemblies. Then cells were incubated for 3 h with MTT solution (0.5 mg/mL in RPMI or BEBM medium). The medium was removed and DMSO (100 μL/well) was added to solubilize formazan crystals. Samples were finally analyzed with absorbance detection at 580 nm on a plate reader (SpectraMax® M2 System, Molecular Devices, UK). The control was performed with cells cultured with medium, without any treatment. Four independent experiments were conducted for CPP-P54 and three independent experiments for CPP-P54/PEG-P54, each with triplicate samples. The half maximal inhibitory concentration ($IC_{50}$) was determined from the dose-response curve. No toxic effect was observed for free peptides.

### 3.2. CPP-P54 suspensions

Self-assemblies of $Arg_x$-P54 (x = 6 to 9) were obtained by nanoprecipitation and the biological activity of these suspensions was assessed against three different cell lines: a healthy human bronchial epithelium cell line (BEAS-2B) and two human lung cancer cell lines (A549 and NCI-H460).

First of all, the influence of the peptide length on the cytotoxic activity was investigated in a human bronchial epithelium cell line (BEAS-2B) and the results were compared with the free P54. The cell viability assay performed using a MTT assay after 24 hours of treatment with various concentrations showed no significant difference between CPP-P54 suspensions and free P54. All the IC50 values were in the same range and no tendency was observed in function of the peptide length.

The same experiments were then performed on two different non-small-cell lung cancer cells lines: A549, an adenocarcinomic human alveolar basal epithelial cell line and NCI-H460, a large-cell carcinoma line. Viability assay performed on A549 showed that the peptide length had an impact on the toxicity: an increase of the peptide length resulted in increased the $Arg_x$-P54 suspension toxicities. In fact, $Arg_9$-P54 was more efficient than the other suspensions. However, no statistically significant difference was observed between suspensions and free P54. These results were confirmed on NCI-H460 cells. In fact, the same trend was observed for the peptide length efficacy: $Arg_9$-P54 suspension has shown to be the most efficient (Fig. 4). However, free P54 had a very high $IC_{50}$, which was statistically different from conjugates.

The obtained results are reported in Table 5 below.

Table 5. **Biological** activities of $Arg_x$-P54 suspensions on three cell lines.

| | BEAS 2B healthy cell line | | A549 cancer cell line | | NCI H460 cancer cell line | |
|---|---|---|---|---|---|---|
| | $IC_{50}$ (μm) | SD | $IC_{50}$ (μm) | SD | $IC_{50}$ (μm) | SD |
| $Arg_6$-P54 | 35 | 1.5 | 231 | 1.01 | 46 | 1.3 |
| $Arg_7$-P54 | 21 | 1.1 | 136 | 1.04 | 31 | 1.1 |
| $Arg_8$-P54 | 12 | 1.1 | 111 | 1.02 | 31 | 1.2 |
| $Arg_9$-P54 | 26 | 1.6 | 83 | 1.08 | 24 | 1.1 |
| P54 | 27 | 1.1 | 216 | 1.01 | 215 | 1.2 |

### 3.3. $Arg_9$-P54 / PEG-P54 suspensions

In this part, all the results were expressed in function of the P54 concentration, knowing that the molar ratio between $Arg_9$ and P54 is 1/1, as for PEG-P54 conjugate.

Viability assays were performed on healthy cell line (BEAS-2B cells) and on lung cancer cells (NCI-H460). Interestingly, PEG-P54 suspension (without $Arg_9$) had an effect on cell viability with an $IC_{50}$ close to $Arg_9$-P54 (31 μM for PEG-P54 and 26 μM for $Arg_9$-P54). Moreover, the incorporation of PEG-P54 in the structure of self-assemblies had no impact on the cytotoxicity.

On NCI-H460, the presence of PEG had a significant impact on the IC50 values: higher IC50 was obtained with a higher amount of PEG, assuming that PEG could hide CPP, causing a decrease of the biological activity45-48 (Fig. 5). In fact, PEG could shield the cell penetrating peptide feature of $Arg_9$-P54, resulting in a decrease of cell internalization. It is important to note that no toxicity was observed for PEG-P54 alone in this range of tested concentration and the highest $IC_{50}$ was obtained for the suspension containing the highest amount of PEG ($Arg_9$-P54/PEG-P54: 1/2). Moreover, all the results obtained for $Arg_9$-P54/PEG-P54 suspensions were statistically significant compared to free P54.

The obtained results are reported in Table 6 below.

Table 6. **Biological** activities of $Arg_9$-P54 / PEG-P54 suspensions on two cell lines.

| | BEAS 2B healthy cell line | | NCI H460 cancer cell line | |
|---|---|---|---|---|
| | $IC_{50}$ (μm) | SD | $IC_{50}$ (μm) | SD |
| $Arg_9$-P54 / PEG-P54 10/1 | 19 | 1.0 | 34 | 1.1 |
| $Arg_9$-P54 / PEG-P54 2/1 | 20 | 1.0 | 53 | 1.2 |
| $Arg_9$-P54 / PEG-P54 1/1 | 25 | 1.0 | 77 | 1.3 |
| $Arg_9$-P54 / PEG-P54 1/2 | 25 | 1.0 | 105 | 1.6 |
| PEG-P54 | 31 | 1.3 | / | / |
| $Arg_9$-P54 | 26 | 1.6 | 24 | 1.1 |

### *4. In vivo* efficacy

Lung cancer was induced in nude mice by subcutaneous injection of lung cancer cells (NCI-H460) in the left lung of xenograft mice model [Hureaux et al., Rev. Mal. Respir. 2015, 32, A96].

- animals:

Female nude mice Rj: NMRI-Foxn1 nu/nu of 7 weeks (JANVIER LABS, Saint Berthevin, France) were housed in the Angers University Hospital animal facility. They were provided with food and water and followed a regular 12-hour day/night cycle. After a week of acclimatization, the animals were used for percutaneous intrapulmonary lung cell cancer administration and intratracheal administration. Animal experiments were performed while respecting the ethics and regulation of animal experimentation (authorization no. 201805281630885).

- Preparation of the Matrigel® solution:

Matrigel® is a basement membrane matrix preparation extracted from Engelbreth-Holm-Swarm mouse sarcoma (ECM Gel from Engelbreth-Holm-Swarm murine sarcoma, Sigma, E6909). It was mixed with the cell suspension in order to improve their attachment to the lung tissue and favor the formation of a mass. The received product was stocked at -80°C, and placed at 4 °C, 24 hours before its use. The received Matrigel® solution (with an initial concentration of 8-12 mg of protein/mL) was diluted 1/3 v/v in HBSS with calcium and magnesium, before mixing with cells.

- Preparation of cell suspension for injection:

The human lung cancer cell line NCI-H460 was chosen to induce primary human lung cancer in nude mice. The cells received at least two passages between thawing and injection (same media and multiplication time mentioned before were respected). 24 h before the injection of the tumor cells, the Matrigel® and the HBSS were stored at 4 °C, the Eppendorfs, the tips of pipettes and the syringes were stored at -20 °C in order to avoid any Matrigel® gelification before the injection. On the day of injection, 35 μL of $10^6$ NCI-H460 cells in HBSS with calcium and magnesium was mixed with 35 μL of a solution of Matrigel® diluted as previously described in cold HBSS with calcium and magnesium. The mixing was performed in an ice bath at 4°C so to keep the Matrigel® in a liquid form. The mice were anesthetized by a continuous air flow (0.5-0.8 L/min) and 20 % isoflurane, placed on their ventral side on a heating support to preserve their corporal temperature. The cell suspension prepared was slowly injected (7-10 seconds to inject 70 μL in order to allow the gelification of the Matrigel® inside the lung) percutaneously using a syringe into the left lateral thorax, 3 mm above the line separating the lung from the liver. The needle was inserted 4 mm into the thorax and was quickly removed after the injection. Once the injection done, the mouse was waked up from the anesthesia and observed until total recovery. The mice weight, their behavior, respiratory rate and the response to normal stimulus were regularly checked.

The presence of the tumor was confirmed via MRI: 9 days after the injection of cancerous cells, MR imaging (MRI) was performed to identify the presence of lung tumors in the mice. MRI was performed using the PRISM MRI facility (Biogenouest, Univ Rennes, Univ Angers, INRAE, CNRS, France) 7T scanner (Biospec 70/20 Avance III, Bruker Wissembourg, France) equipped with BGA12S gradient system (675 mT/m) and a 35 mm diameter resonator. For the duration of all MR experiments under isoflurane anesthesia, animal temperature was regulated by a heated water-circulating system. After a set of scout images, retrospectively gated $T_1$-weighted images were acquired to follow the volume of tumours using an IntraGate® FLASH 3D sequence (TR = 110 ms; TE = 1.6 ms; $\alpha$ = 50°; FOV = 25 × 25 mm;

matrix = 192 × 192; nine slices of 0.7 mm).

The mice with lung tumors were homogenously distributed into four groups, three treated by nebulization of 3 different self-assemblies: $Arg_9$-P54/PEG-P54 (10/1 molar ratio), $Arg_9$-P54 and PEG-P54 at a concentration of 1 mM and a control group nebulized with physiological water.

First of all, in order to study the tolerance of mice to the different treatments, their weight was followed during the whole experiment (Fig. 6).

The mice form the different four groups showed no significant loss of weight proving a good tolerance to treatment. Meanwhile, it is important to note that the group treated with $Arg_9$-P54 self-assemblies showed clear suffering signs after nebulization: heavy breathing and coughing. This was not the case for the group treated with $Arg_9$-P54/PEG-P54.

Two nebulizations have been performed at day 9 and day 12 after the injection of the tumor cells, via intratracheal route 30. In order to regulate their osmolarity before the nebulization, an adequate volume of a 9% NaCl solution was added to the different formulations. A final osmolarity of 280-300 mOsm was suitable for a pulmonary administration.

Mice were anesthetized by a continuous air flow (0.5-0.8 L/min) and 20 % isoflurane. The mice were fixed on an inclined support: it allowed visualizing the trachea of the mouse once its moth is opened and the tongue is moved away from the field of vision. A laryngoscope was used to open the animal's mouth and to take the tongue out of the way of vision. Once the trachea is clear, the syringe was introduced between the two vocal cords, and the injection was performed.

In order to create an aerosol and mimic the nebulizer, a micro-sprayer system was used (High pressure syringe), allowing to create microdrops of the suspension, dispersing into the two lungs via an aerosol. The volume nebulized was of 50 μL.

When the nebulization is over, the animal was waked up on a heating support and his behavior was carefully controlled until perfect recovery.

Finally, the tumor size evolution was analyzed by MRI at different days: day 9, 12 ,15, 21, 23, 25 and 27 after the injection of the tumor cells.

First, the tumors volumes were measured from the MRI images. They were calculated from manually drawn ROI on each slice, the area being then multiplied by the slice thickness.

These tumor volume growth curves are then fitted by the method of least squares, with an exponential function and the times constant of exponential converted into a doubling time value as follows:

$$V(t) = V_0 e^{\alpha t}$$

Where Vo is the volume at $t_0$ and $\alpha$: fraction of proliferative cells.

If $V'(t') = V_0 e^{\alpha t'}$, t' being chosen as V'=2V then $V_0 e^{\alpha t'} = 2V_0 e^{at}$, so $ln(2) = ln\frac{e^{\alpha t'}}{e^{\alpha t}}$ and(t - t') = (ln(2))/$\alpha$. (t-t') being the doubling time.

In order to test if the data follows a normal law, two tests were applied: Shapiro-Wilk test and Kolmogorov-Smirnov test. Then a t test, with the correction of Welch, was applied to compare each treated group to the control group. Differences were considered as significant if the p value was $< 0.05$.

The results, which are represented in Figure 7, showed that compared to the control group, the treatment by nebulization of $Arg_9$-P54/PEG-P54 self-assemblies (Fig. 7A) or of $Arg_9$-P54 self-assemblies (Fig. 7B) lengthened significantly ($p < 0.05$) the doubling time, and so slowed down the tumor development. On the other hand, treatment by nebulization of PEG-P54 self-assemblies (Fig. 7C) had no significant effect on the doubling time compared to the control group.

To conclude, in order to combine a significant anticancer effect and a good tolerance of treatment by the mice, the self-assemblies composed of $Arg_9$-P54/PEG-P54 will be really promising.

## Claims

1. A self-assembling product comprising a co-assembly of a CPP-ferrocifen conjugate, and a PEG-ferrocifen conjugate, wherein:

    - the ferrocifen is a compound of the following formula (I):

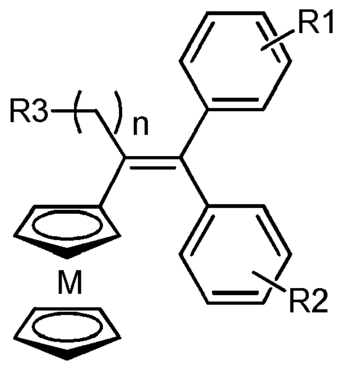

(I)

or a pharmaceutically acceptable salt or solvate thereof,
in which:

- M is Fe (iron), Ru (ruthenium) or Os (osmium), preferably Fe,
- n is an integer comprised between 1 and 8,
- R1 and R2 are, independently from each other, H, $CF_3$, CN, $OR^4$ or $NR^5R^6$, and
- R3 is $CO_2R^7$, $OR^8$ or $NR^9R^{10}$,

wherein:

- $R^4$ is H, $(C_1\text{-}C_6)$alkyl, -CO-$(C_1\text{-}C_6)$alkyl or $-(CH_2)_mNR^{11}R^{12}$,
- $R^5$, $R^6$, $R^{11}$ and $R^{12}$ are, independently from one another, H, $(C_1\text{-}C_6)$alkyl or -CO-$(C_1\text{-}C_6)$alkyl,
- $R^7$ is H or $(C_1\text{-}C_6)$alkyl,
- $R^8$ is $-CO-(CH_2)_pCO_2R^{13}$
- $R^9$ is H or $(C_1\text{-}C_6)$alkyl,
- $R^{10}$ is $-CO-(CH_2)_qCO_2R^{14}$,
- $R^{13}$ and $R^{14}$ are, independently from one another, H or $(C_1\text{-}C_6)$alkyl, and
- m, p and q are, independently from one another, integers comprised between 1 and 8;

▪ CPP is a hydrophilic cell-penetrating peptide, covalently bonded to the R3 group of the ferrocifen by means of an amide (-C(O)NH-) bond; and
▪ PEG is a polyethylene glycol chain, covalently bonded to the R3 group of the ferrocifen by means of an ester (-C(O)O-) bond;
and wherein the CPP-ferrocifen / PEG-ferrocifen molar ratio is comprised between 100 / 1 and 1/1, preferably between 50/1 and 2/1, notably between 20/1 and 5/1, in particular between 15/1 and 7/1, more preferably between 12/1 and 8/1, for example, 10/1.

2. The self-assembling product according to claim 1, wherein the ferrocifen has the following formula (Ia) or preferably (Ia-Fe):

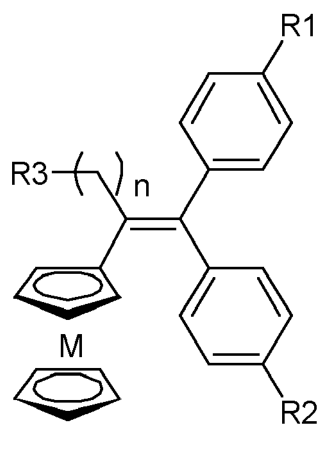

(Ia),

preferably

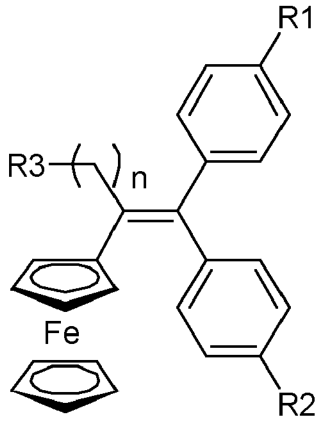

(Ia-Fe).

**3.** The self-assembling product according to claim 1 or 2, wherein n is comprised between 2 and 6, notably between 2 and 5, and wherein R1 and R2 are, independently from each other, H, $OR^4$ or $NR^5R^6$.

**4.** The self-assembling product according to any one of claims 1 to 3, wherein R1 and R2 are identical, preferably R1 and R2 are OH.

**5.** The self-assembling product according to any one of claims 1 to 4, wherein R3 is $CO_2R^7$, $OR^8$ or $NR^9R^{10}$, and wherein:

- $R^7$ is H,
- $R^8$ is $-CO-(CH_2)_pCO_2R^{13}$, wherein $R^{13}$ is H,
- $R^9$ is H or $(C_1-C_6)$alkyl, preferably H,
- $R^{10}$ is $-CO-(CH_2)_qCO_2R^{14}$, wherein $R^{14}$ is H, and
- p and q are, independently from one another, integers comprised between 1 and 6, notably between 1 and 4, preferably between 2 and 3.

**6.** The self-assembling product according to any one of claims 1 to 5, wherein the ferrocifen is selected from the group consisting of:

| | |
|---|---|
| 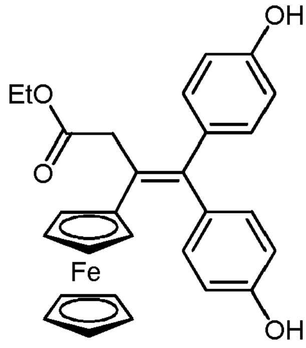<br>**P64** | 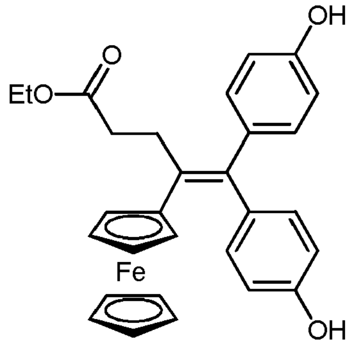<br>**P49** |
| 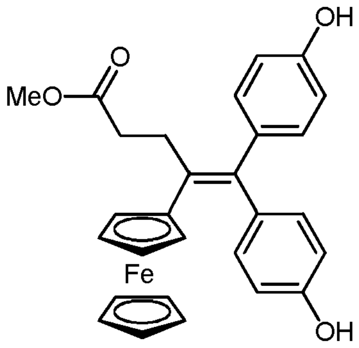<br>**P189** | 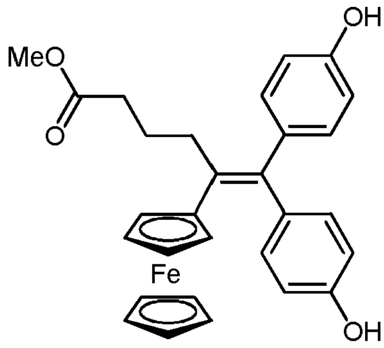<br>**P188** |
| 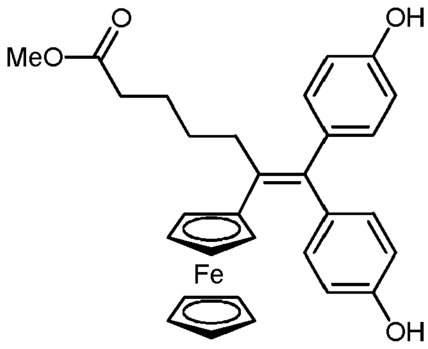<br>**P504** | 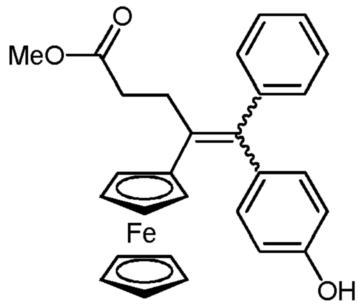<br>**P680** |

| | |
|---|---|
| 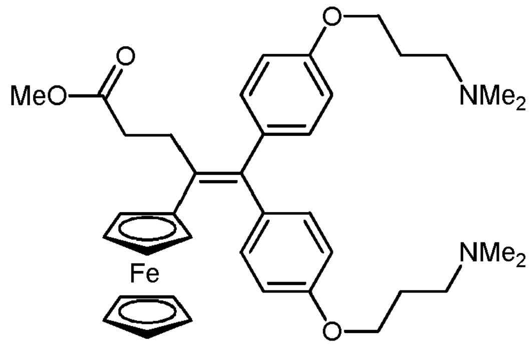<br>**P632** | 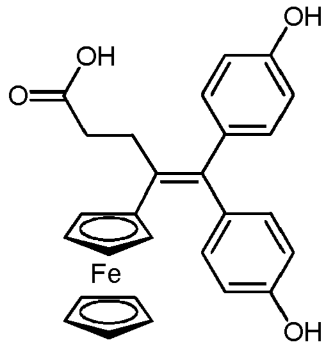<br>**P54** |
| 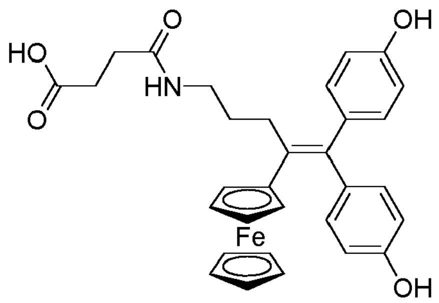<br>**P819** | |

and the pharmaceutically acceptable salts and/or solvates thereof.

**7.** The self-assembling product according to any one of claims 1 to 6, wherein the CPP is a polycationic peptide, comprising between 5 and 15, notably between 5 and 12, preferably between 6 and 9 positively charged amino acids, such as lysine and arginine.

**8.** The self-assembling product according to claim 7, wherein the CPP is a polyarginine of the following formula ($Arg_x$):

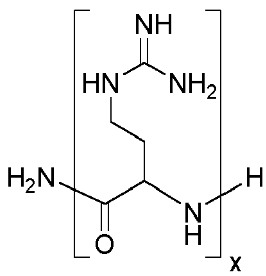
($Arg_x$),

wherein x is an integer comprised between 5 and 15, notably between 5 and 12, preferably between 6 and 9.

**9.** The self-assembling product according to any one of claims 1 to 8, wherein the PEG moiety is of the following formula ($PEG_y$):

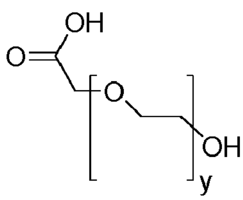
($PEG_y$),

wherein y is an integer comprised between 20 and 200, notably between 30 and 100, preferably between 40 and 50, for example, 44.

**10.** The self-assembling product according to any one of claims 1 to 9, for use as a drug.

**11.** The self-assembling product according to any one of claims 1 to 9, for use as a drug in the treatment of cancer.

**12.** The self-assembling product for use according to claim 11, wherein the cancer is selected from the group consisting of gliomas, melanomas, retinoblastomas, breast cancers such as non-hormone-dependent breast cancers, prostate cancers, lung cancers such as non-small-cell lung cancers, ovarian cancers, esophageal cancers, liver cancers, pancreatic cancers, head and neck cancers, colon cancers and kidney cancers.

**13.** The self-assembling product for use according to claim 12, wherein the cancer is a lung cancer such as a non-small-cell lung cancer, and the self-assembling product is preferably administered by intrapulmonary route via nebulization.

**14.** A method for preparing the self-assembling product as defined in any one of claims 1 to 9 by co-nanoprecipitation of the CPP-ferrocifen and PEG-ferrocifen conjugates, comprising the steps of:

- solubilizing the conjugates in a polar organic solvent in a CPP-ferrocifen / PEG-ferrocifen molar ratio comprised between 100 / 1 and 1/1, preferably between 50/1 and 2/1, notably between 20/1 and 5/1, in particular between 15/1 and 7/1, more preferably between 12/1 and 8/1, for example, 10/1;
- adding the obtained organic mixture dropwise to a volume of water under stirring, the water / polar organic solvent volume ratio being comprised between 10/1 and 2/1, for example between 8/1 and 2/1, notably between 6/1 and 3/1, in particular between 5/1 and 3/1, for example 4/1; and
- evaporating the polar organic solvent.

**15.** A PEG-ferrocifen conjugate, wherein:

- the PEG chain is of the following formula ($PEG_y$):

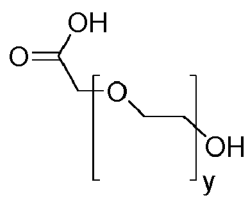

($PEG_y$),

wherein y is an integer comprised between 20 and 200, notably between 30 and 100, preferably between 40 and 50, for example, 44, and
- the ferrocifen is as defined in any one of claims 1 to 6, and is covalently bonded to the PEG chain by means of an ester bond via its R3 group.

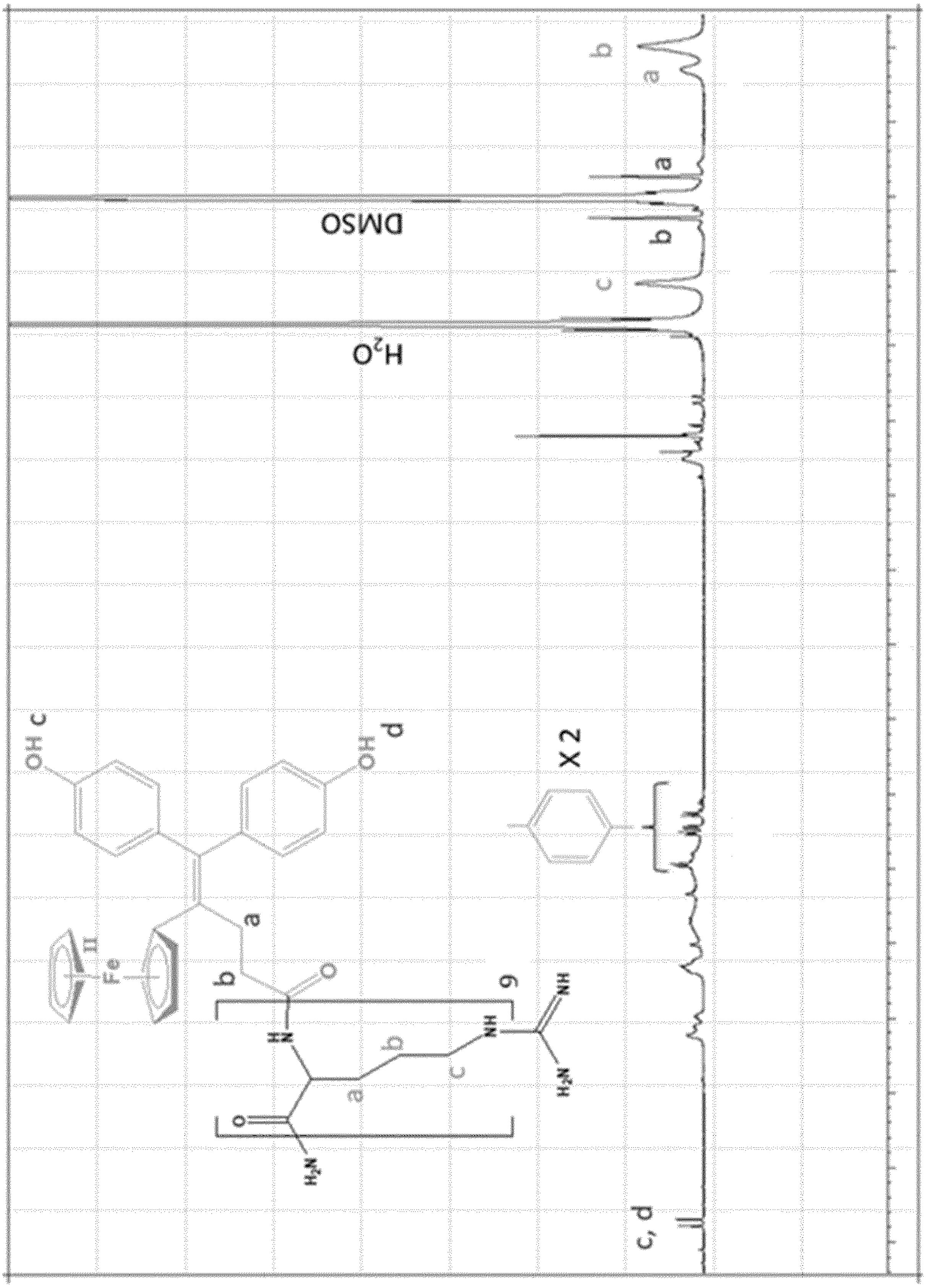


Fig. 1A

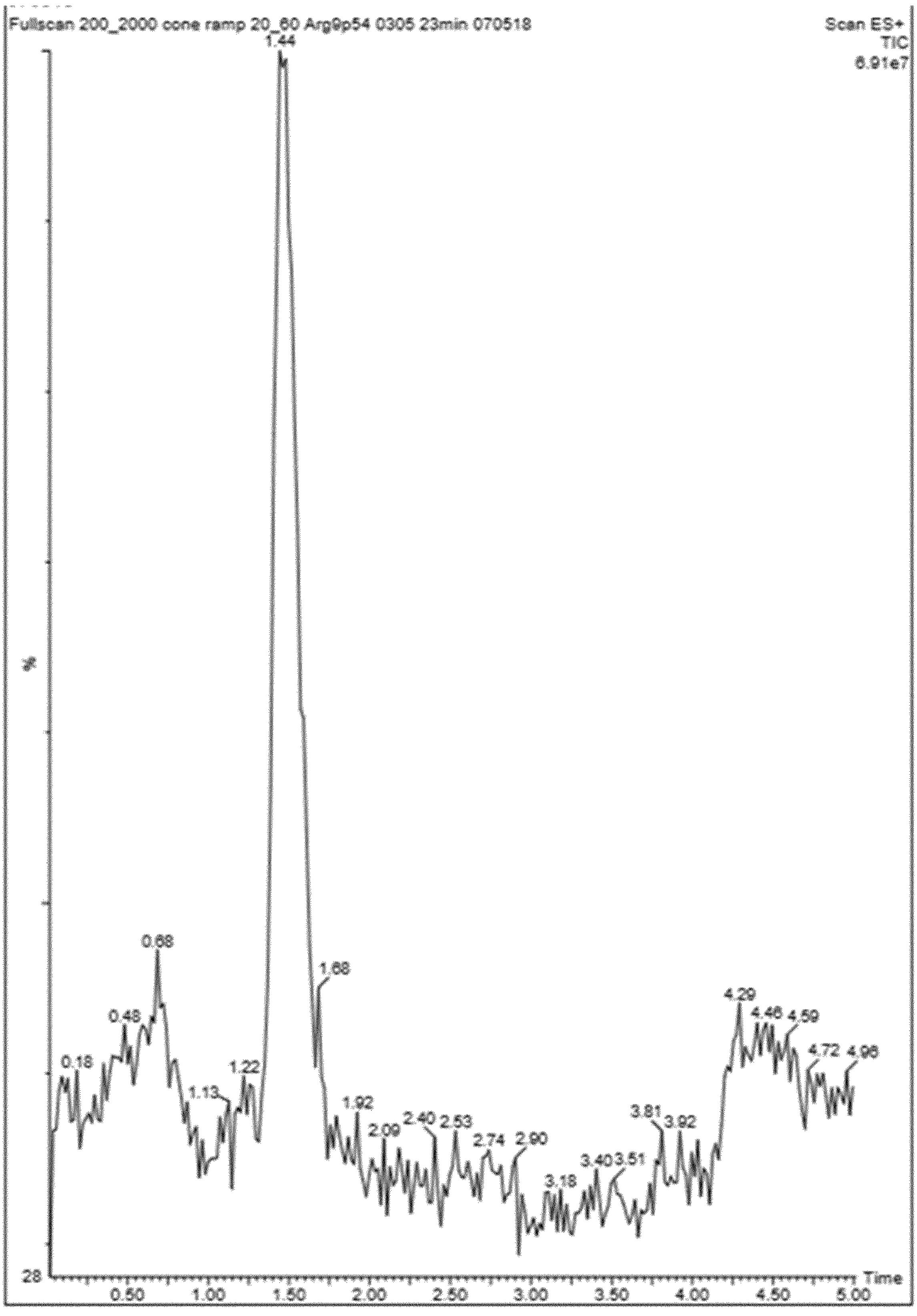
Fullscan 200_2000 cone ramp 20_60 Arg9p54 0305 23min 070518
Scan ES+
TIC
6.91e7
1.44
0.68
1.68
0.48
0.18
1.22
1.13
1.92
2.09
2.40
2.53
2.74
2.90
3.18
3.40
3.51
3.81
3.92
4.29
4.46
4.59
4.72
4.96
%
28
0.50
1.00
1.50
2.00
2.50
3.00
3.50
4.00
4.50
5.00
Time

Fig. 1B

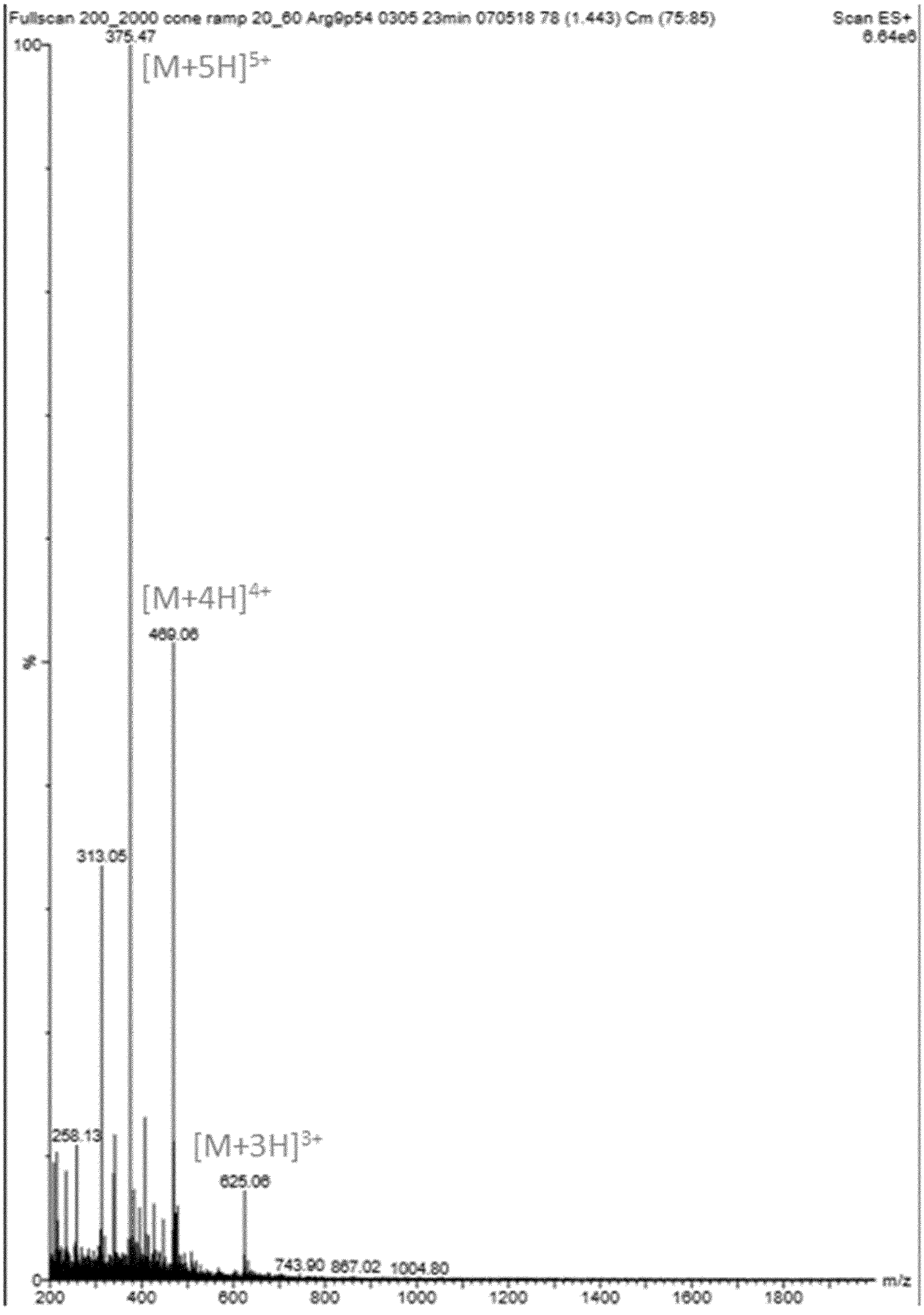
Fullscan 200_2000 cone ramp 20_60 Arg9p54 0305 23min 070518 78 (1.443) Cm (75:85)
Scan ES+
6.64e6
100
%
0
375.47
[M+5H]5+
[M+4H]4+
469.06
313.05
258.13
[M+3H]3+
625.06
743.90
867.02
1004.80
m/z
200
400
600
800
1000
1200
1400
1600
1800

Fig. 1C

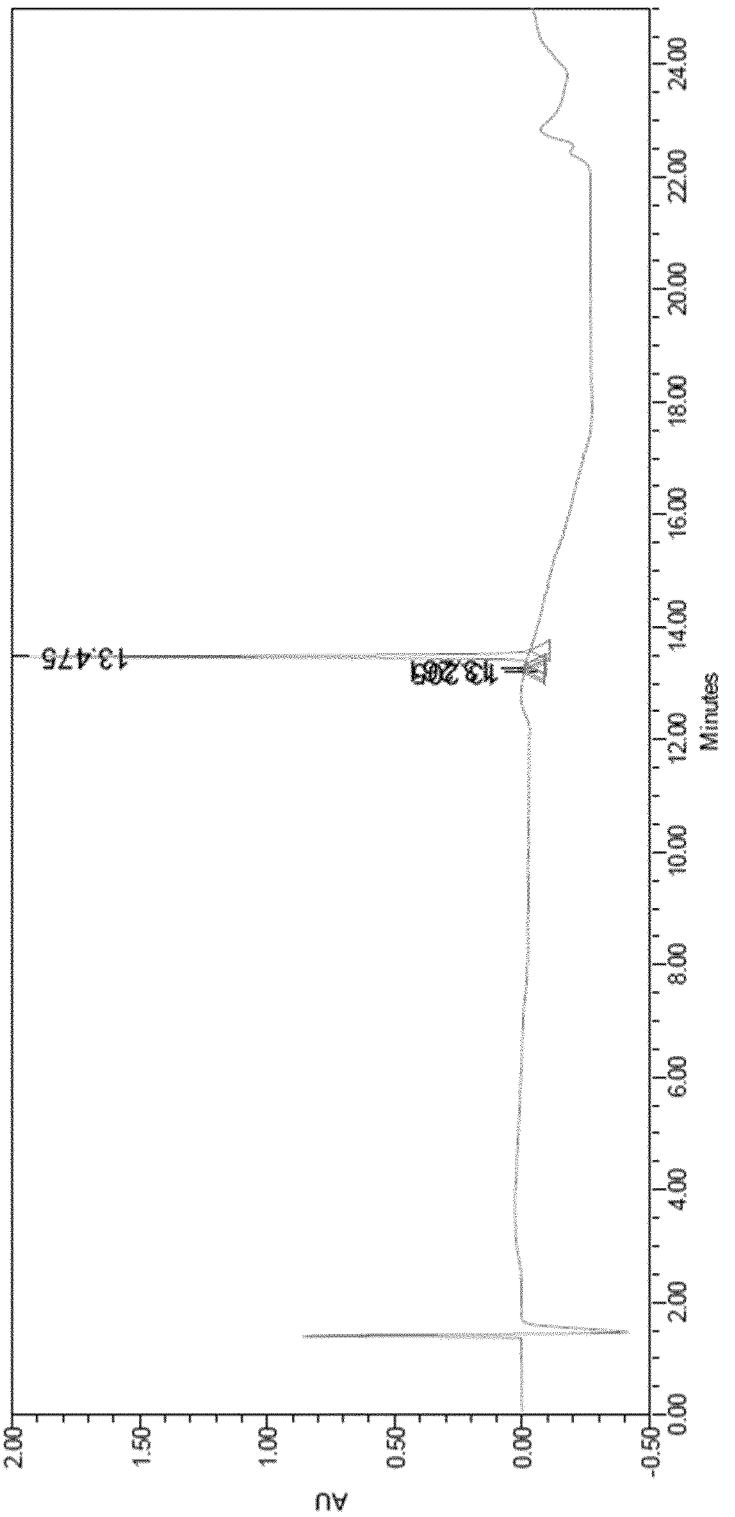
13.475
13.265
Minutes
AU
0.00
2.00
4.00
6.00
8.00
10.00
12.00
14.00
16.00
18.00
20.00
22.00
24.00
2.00
1.50
1.00
0.50
0.00
-0.50

Fig. 1D

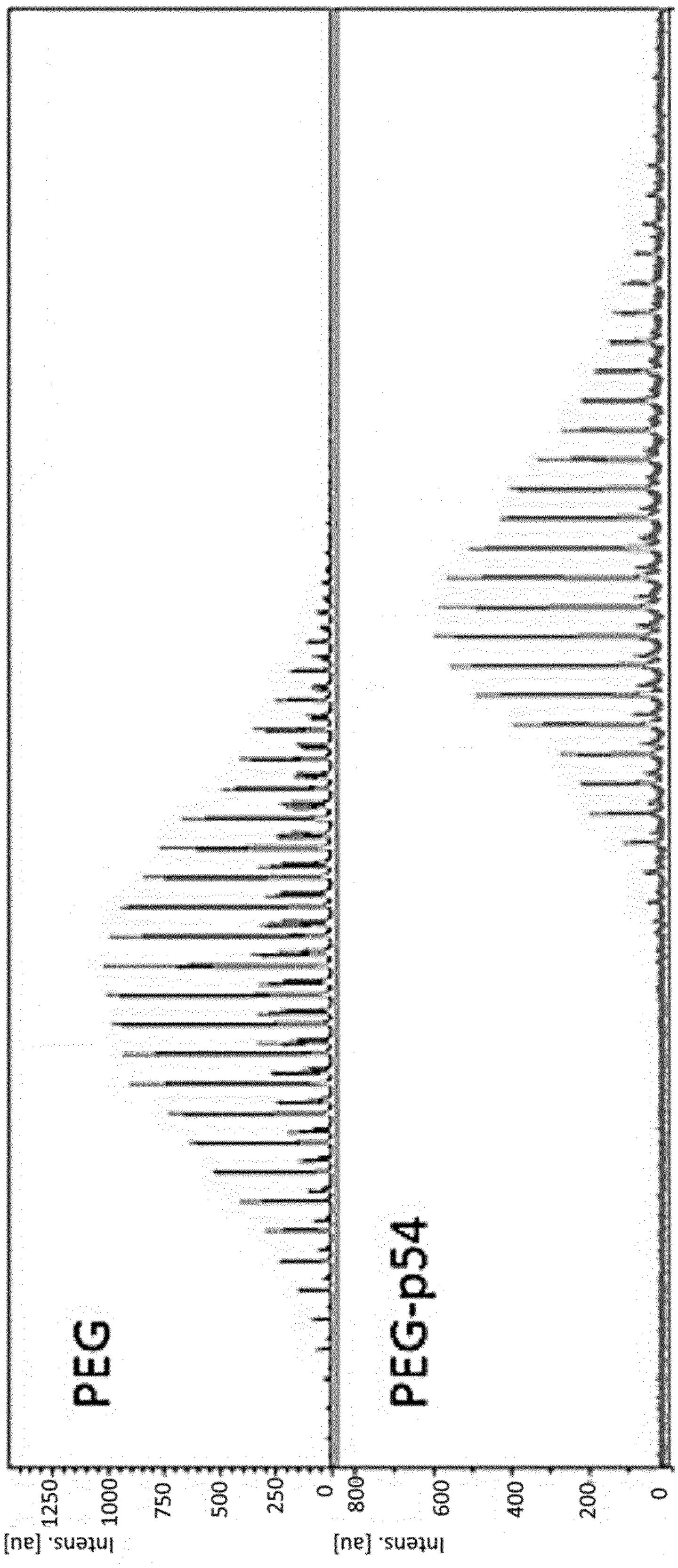
PEG
PEG-p54
Intens. [au]
1250
1000
750
500
250
0
Intens. [au]
800
600
400
200
0

Fig. 2A

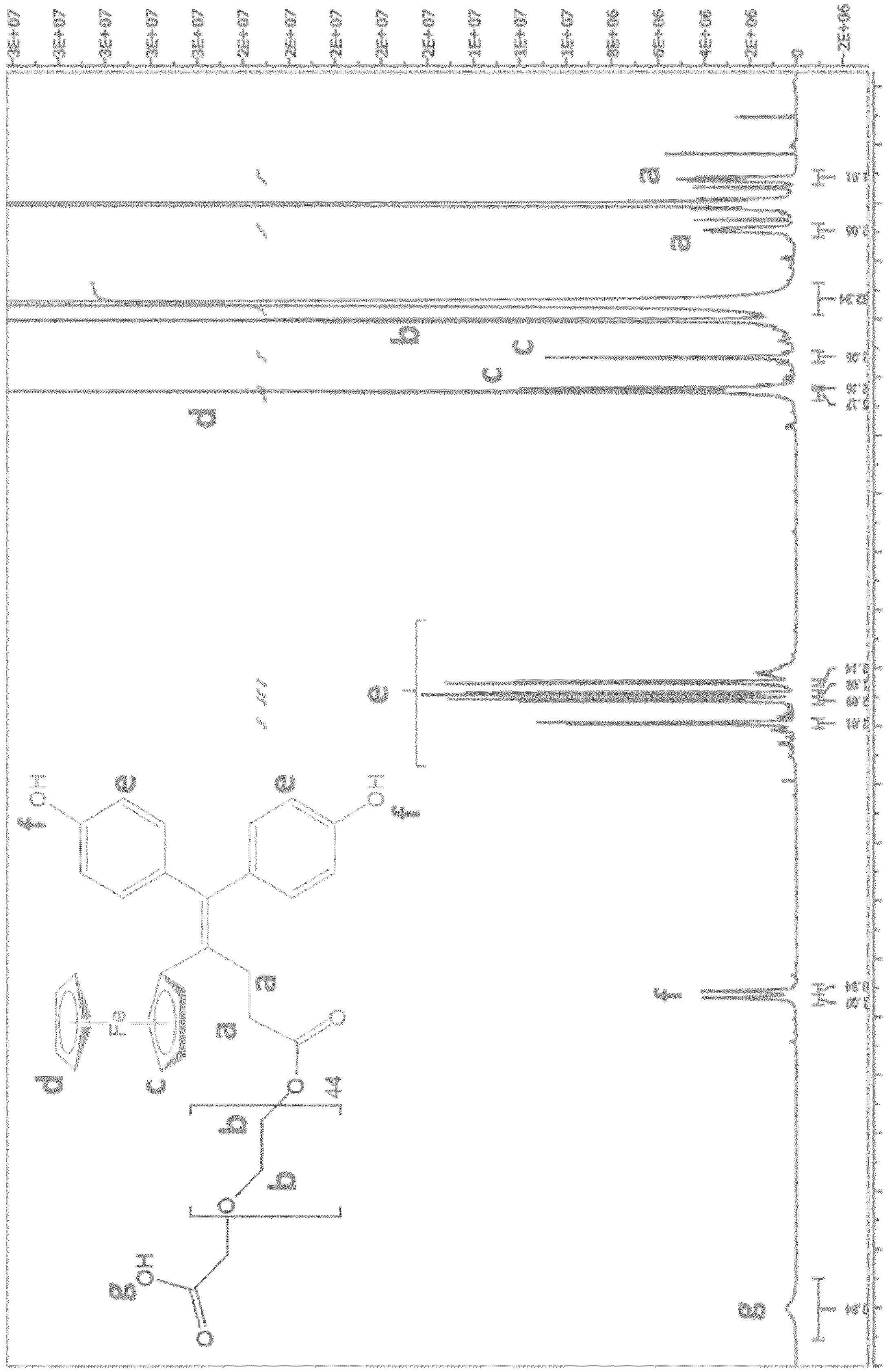

Fig. 2B

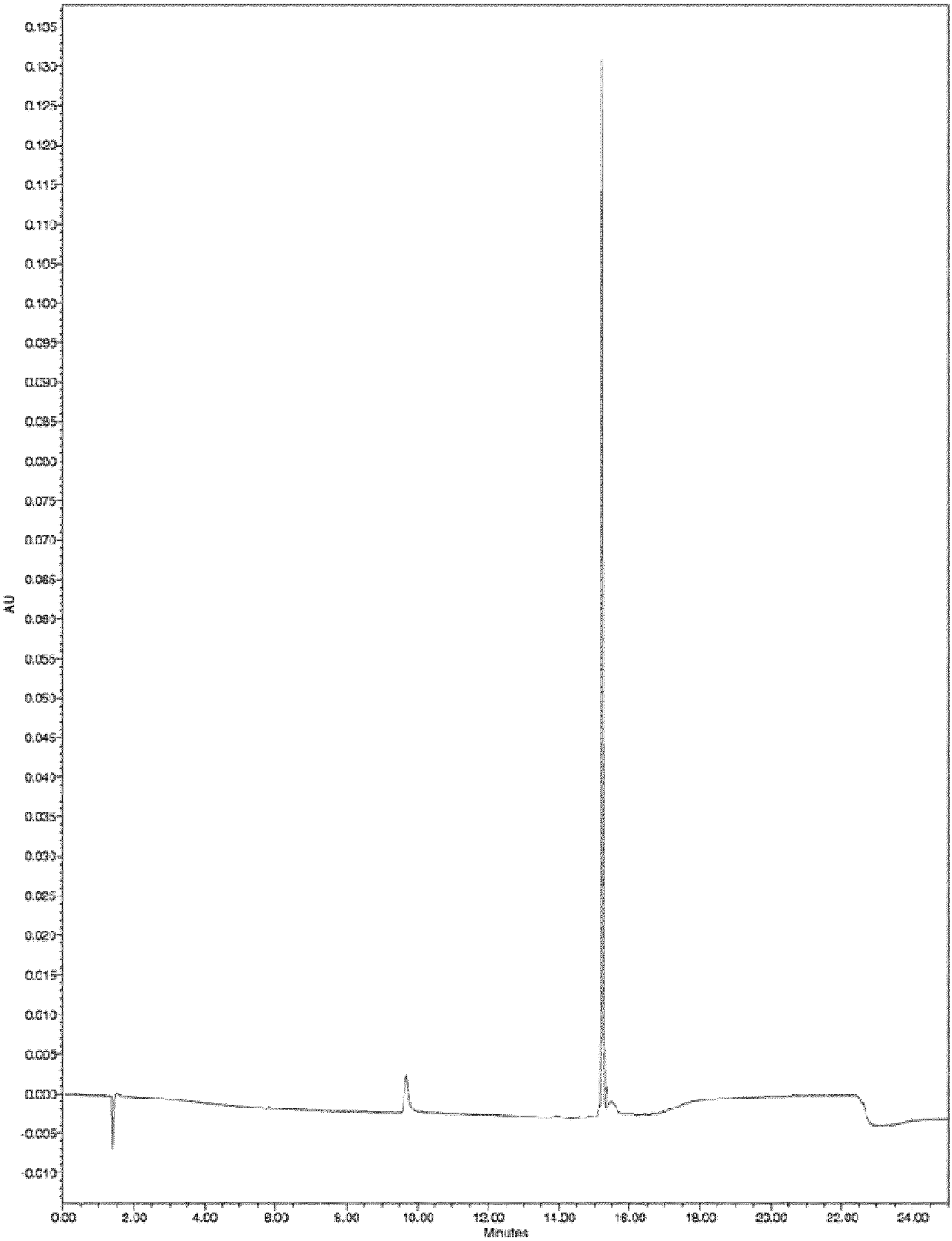
0.135
0.130
0.125
0.120
0.115
0.110
0.105
0.100
0.095
0.090
0.085
0.080
0.075
0.070
0.065
0.060
0.055
0.050
0.045
0.040
0.035
0.030
0.025
0.020
0.015
0.010
0.005
0.000
-0.005
-0.010
AU
0.00
2.00
4.00
6.00
8.00
10.00
12.00
14.00
16.00
18.00
20.00
22.00
24.00
Minutes

Fig. 2C

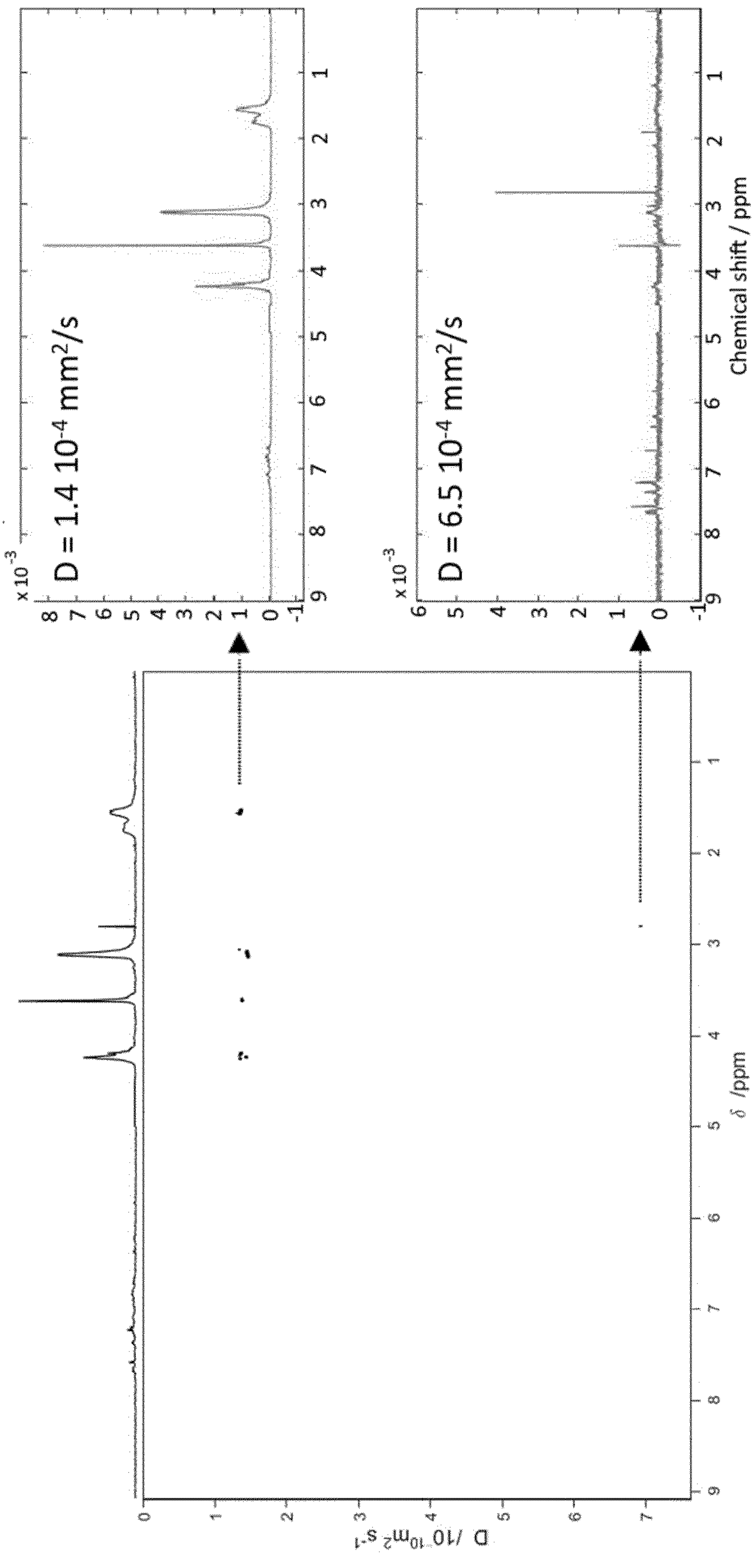


**Fig. 3**

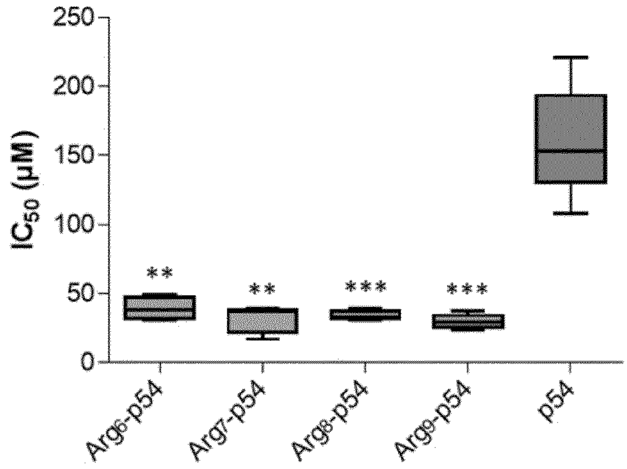


Fig. 4

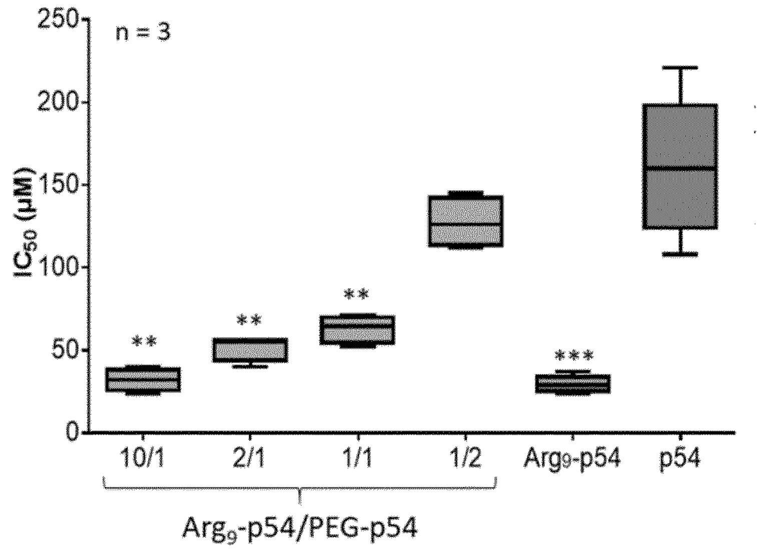


Fig. 5

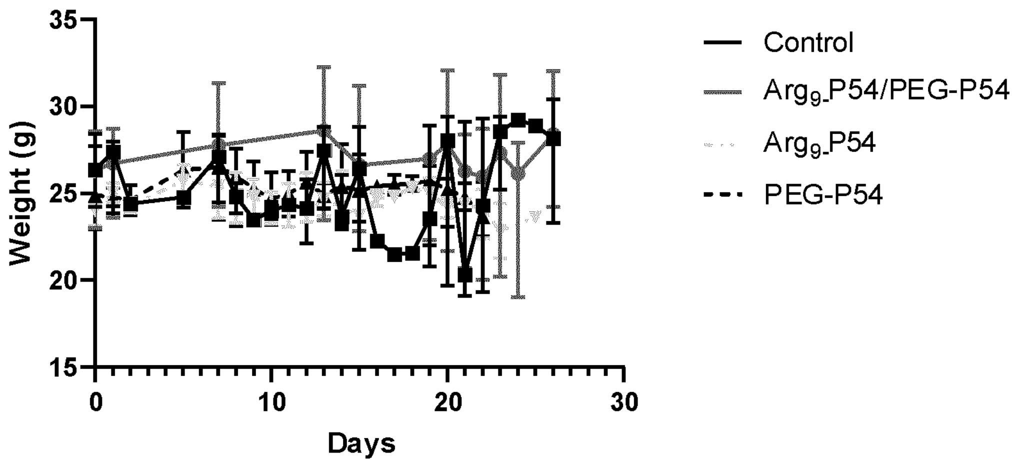


Fig.6

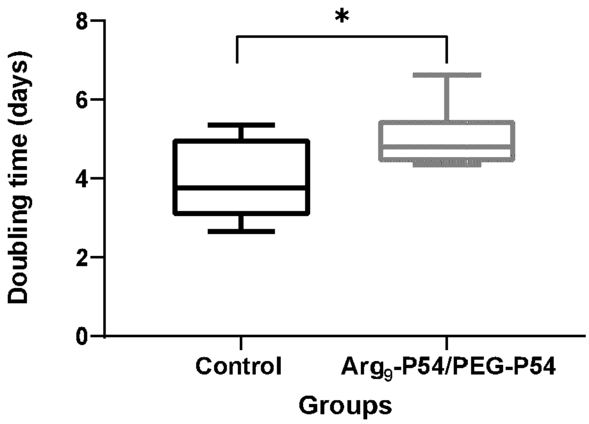


Fig. 7A

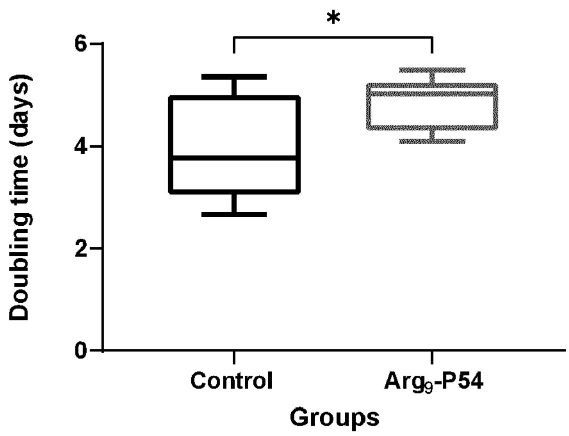


Fig. 7B

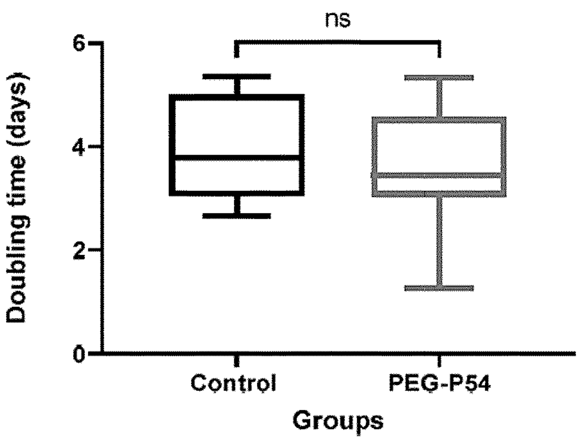


Fig. 7C

Europäisches Patentamt
European Patent Office
Office européen des brevets

# EUROPEAN SEARCH REPORT

Application Number
EP 22 30 5559

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | GUYON LÉNA ET AL: "Importance of Combining Advanced Particle Size Analysis Techniques To Characterize Cell-Penetrating Peptide-Ferrocifen Self-Assemblies", JOURNAL OF PHYSICAL CHEMISTRY LETTERS, vol. 10, no. 21, 14 October 2019 (2019-10-14), pages 6613-6620, XP055961319, US ISSN: 1948-7185, DOI: 10.1021/acs.jpclett.9b01493 | 1-5,7-14 | INV. A61K47/60 A61K47/64 A61P35/00 |
| Y | * whole document * | 15 | |
| A | | 6 | |
| X | & GUYON LÉNA ET AL: "Supporting information - The importance to combine advanced particle size analysis techniques to characterize cell penetrating peptide-ferrocifen self-assemblies", J. PHYS. CHEM. LETT., vol. 10, no. 21, 14 October 2019 (2019-10-14), pages s1-s19, XP055961626, Retrieved from the Internet: URL:https://pubs.acs.org/doi/suppl/10.1021/acs.jpclett.9b01493/suppl_file/jz9b01493_si_001.pdf> | 1-5,7-14 | TECHNICAL FIELDS SEARCHED (IPC) |
| | | | A61K |
| Y | * figure S1 * | 15 | |
| A | ----- | 6 | |

-/--

The present search report has been drawn up for all claims

1

| Place of search | Date of completion of the search | Examiner |
|---|---|---|
| The Hague | 19 September 2022 | Birikaki, Lemonia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 30 5559

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | AL-AMILI MAJDI ET AL: "Self-Assembled Micelles of Amphiphilic PEGylated Drugs for Cancer Treatment", CURRENT DRUG TARGETS, vol. 22, no. 8, 31 December 2020 (2020-12-31), pages 870-881, XP055961891, US ISSN: 1389-4501, DOI: 10.2174/1389450122666201231130702 * whole document and in particular the abstract * ----- | 15 | |
| | | | TECHNICAL FIELDS SEARCHED (IPC) |

1

The present search report has been drawn up for all claims

| Place of search | Date of completion of the search | Examiner |
|---|---|---|
| The Hague | 19 September 2022 | Birikaki, Lemonia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **GUYON et al.** *Nano Res,* 2018, vol. 11 (5), 2315-2335
- **LINDGREN et al.** *Trends Pharmacol. Sci.,* 2000, vol. 21 (3), 99-103
- **JAOUEN et al.** *Chem. Soc. Rev.,* 2015, vol. 44 (24), 8802-8817
- **VESSIÈRES et al.** *J. Inorg. Biochem.,* 2010, vol. 104 (5), 503-511
- **GUYON et al.** *J. Phys. Chem. Lett.,* 2019, vol. 10, 6613-3320
- **HUREAUX et al.** *Rev. Mal. Respir.,* 2015, vol. 32, A96